# EUROPEAN PATENT APPLICATION

(11) **EP 1 764 414 A1**
(43) Date of publication of application: **21.03.2007**
(21) Application number: 05020311.6
(22) Date of filing: 17.09.2005
(51) Int. Cl.: C12N 15/82, C07K 14/08

(54) **Plant viral particles comprising a plurality of fusion proteins consisting of a plant viral coat protein, a peptide linker and a recombinant protein and use of such plant viral particles for protein purification**

(71) Applicant: Icon Genetics AG, 80333 München (DE)
(72) Inventor: Werner, Stefan, 06110 Halle/Saale (DE); Marillonnet, Sylvestre, 06126 Halle/Saale (DE); Klimyuk, Victor, 06114 Halle/Saale (DE); Gleba, Yuri, 06114 Halle/Saale (DE)
(74) Representative: Hartz, Nikolai

(57) **Abstract**

Viral particles or virus-like particles comprising a plurality of fusion protein molecules, said fusion protein comprising the following fusion protein domains:
(i) a plant viral coat protein,
(ii) a recombinant protein, and
(iii) at least one peptide linker linking said plant viral coat protein and said recombinant protein,
wherein formation of said viral particle does not require free viral coat protein.

### Annex to the application documents - subsequently filed sequences listing

SEQUENCE LISTING

<110>  Icon Genetics AG

<120>  Protein Purification Using Plant Viral Particles

<130>  EAD-13417

<140>  EP 05 020 311.6
<141>  2005-09-17

<160>  36

<170>  PatentIn version 3.1

<210>  1
<211>  38
<212>  DNA
<213>  Artificial Sequence

<220>
<223>  PCR primer

<400>  1
cggagctctt aatttaaaag aagaaaatgt cttacaac                              38


<210>  2
<211>  79
<212>  DNA
<213>  Artificial Sequence

<220>
<223>  PCR primer

<400>  2
tttggtctca tacctgagcc accgcctcct gatccaccgc ctccacttcc tccgcctcct      60

gtagcaggcg cagtagtcc                                                   79


<210>  3
<211>  44
<212>  DNA
<213>  Artificial Sequence

<220>
<223>  PCR primer

<400>  3
cagctagcaa caaacaagaa atgtcttaca acattacaaa cccg                       44


<210>  4
<211>  36
<212>  DNA
<213>  Artificial Sequence

<220>
<223>  PCR primer

<400>  4
gagctctctc gagcatgcta cgcccccaac tgagag                                36

<210> 5
<211> 42
<212> DNA
<213> Artificial Sequence

<220>
<223> PCR primer

<400> 5
gaagacaaag gtgctgctga ggctggaatt acaggcacct gg 42


<210> 6
<211> 44
<212> DNA
<213> Artificial Sequence

<220>
<223> PCR primer

<400> 6
gaagacaatg taacataagt tcctgtaagt gcaccatcgg cgcc 44


<210> 7
<211> 45
<212> DNA
<213> Artificial Sequence

<220>
<223> PCR primer

<400> 7
gaagacaata cagctagagg taatgcagaa agacgttacg tcctg 45


<210> 8
<211> 46
<212> DNA
<213> Artificial Sequence

<220>
<223> PCR primer

<400> 8
ggatccaagg tctcaacctg ctgctgatgg tttcaccttg gtgaag 46


<210> 9
<211> 35
<212> DNA
<213> Artificial Sequence

<220>
<223> PCR primer

<400> 9
tttggtctca gagtgtaacg tctttctgca ttacc 35


<210> 10
<211> 34
<212> DNA

<213>  Artificial Sequence

<220>
<223>  PCR primer

<400>  10
tttggtctca actcttaccg gtcgttacga cagc                                34


<210>  11
<211>  32
<212>  DNA
<213>  Artificial Sequence

<220>
<223>  PCR primer

<400>  11
tttggtctca ccctccaacc gagggcggtg cc                                  32


<210>  12
<211>  32
<212>  DNA
<213>  Artificial Sequence

<220>
<223>  PCR primer

<400>  12
tttggtctca agggtggcct ggaagaataa ct                                  32


<210>  13
<211>  34
<212>  DNA
<213>  Artificial Sequence

<220>
<223>  PCR primer

<400>  13
tttggtctca gtgtccactg ggtgttgatc ctcg                                34


<210>  14
<211>  35
<212>  DNA
<213>  Artificial Sequence

<220>
<223>  PCR primer

<400>  14
tttggtctca acactgactt ccggcaccac cgagg                               35


<210>  15
<211>  37
<212>  DNA
<213>  Artificial Sequence

<220>
<223>  PCR primer

<400>  15
tttggtctca tctaagtgtg gacttccagg cgttggc                                37


<210>  16
<211>  36
<212>  DNA
<213>  Artificial Sequence

<220>
<223>  PCR primer

<400>  16
tttgaagact atagaggaca cgacaccttc accaag                                 36


<210>  17
<211>  33
<212>  DNA
<213>  Artificial Sequence

<220>
<223>  PCR primer

<400>  17
ctagcttgga gtcatccaca gttcgagaaa taa                                    33


<210>  18
<211>  33
<212>  DNA
<213>  Artificial Sequence

<220>
<223>  PCR primer

<400>  18
aagcttattt ctcgaactgt ggatgactcc aag                                    33


<210>  19
<211>  159
<212>  PRT
<213>  Tobacco mosaic virus U1

<400>  19

Met Ser Tyr Ser Ile Thr Thr Pro Ser Gln Phe Val Phe Leu Ser Ser
1               5                   10                  15


Ala Trp Ala Asp Pro Ile Glu Leu Ile Asn Leu Cys Thr Asn Ala Leu
            20                  25                  30


Gly Asn Gln Phe Gln Thr Gln Gln Ala Arg Thr Val Val Gln Arg Gln
        35                  40                  45


Phe Ser Glu Val Trp Lys Pro Ser Pro Gln Val Thr Val Arg Phe Pro
    50                  55                  60

Asp Ser Asp Phe Lys Val Tyr Arg Tyr Asn Ala Val Leu Asp Pro Leu
65                  70                  75                  80

Val Thr Ala Leu Leu Gly Ala Phe Asp Thr Arg Asn Arg Ile Ile Glu
                85                  90                  95

Val Glu Asn Gln Ala Asn Pro Thr Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Ile Val Glu Leu Ile Arg Gly Thr Gly Ser Tyr Asn Arg Ser Ser
    130                 135                 140

Phe Glu Ser Ser Ser Gly Leu Val Trp Thr Ser Gly Pro Ala Thr
145                 150                 155

<210>  20
<211>  159
<212>  PRT
<213>  Tobamovirus OM

<400>  20

Met Ser Tyr Ser Ile Thr Thr Pro Ser Gln Phe Val Phe Leu Ser Ser
1               5                   10                  15

Ala Trp Ala Asp Pro Ile Glu Leu Ile Asn Leu Cys Thr Asn Ala Leu
            20                  25                  30

Gly Asn Gln Phe Gln Thr Gln Gln Ala Arg Thr Val Val Gln Arg Gln
        35                  40                  45

Phe Ser Glu Val Trp Lys Pro Ser Pro Gln Val Thr Val Arg Phe Pro
    50                  55                  60

Asp Ser Asp Phe Lys Val Tyr Arg Tyr Asn Ala Val Leu Asp Pro Leu
65                  70                  75                  80

Val Thr Ala Leu Leu Gly Ala Phe Asp Thr Arg Asn Arg Ile Ile Glu
                85                  90                  95

Val Glu Asn Gln Ala Asn Pro Thr Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Val Val Glu Leu Ile Arg Gly Thr Gly Ser Tyr Asn Arg Ser Ser

130                 135                 140

Phe Glu Ser Ser Ser Gly Leu Val Trp Asn Ser Gly Pro Ala Thr
145                 150                 155

<210>  21
<211>  159
<212>  PRT
<213>  Tobamovirus ToMV

<400>  21

Met Ser Tyr Ser Ile Thr Ser Pro Ser Gln Phe Val Phe Leu Ser Ser
1               5                   10                  15

Val Trp Ala Asp Pro Ile Glu Leu Leu Asn Val Cys Thr Asn Ser Leu
            20                  25                  30

Gly Asn Gln Phe Gln Thr Gln Gln Ala Arg Thr Thr Val Gln Gln Gln
        35                  40                  45

Phe Ser Glu Val Trp Lys Pro Phe Pro Gln Ser Thr Val Arg Phe Pro
    50                  55                  60

Gly Asp Val Tyr Lys Val Tyr Arg Tyr Asn Ala Val Leu Asp Pro Leu
65                  70                  75                  80

Ile Thr Ala Leu Leu Gly Ala Phe Asp Thr Arg Asn Arg Ile Ile Glu
                85                  90                  95

Val Glu Asn Gln Gln Ser Pro Thr Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Val Asn Glu Leu Val Arg Gly Thr Gly Leu Tyr Asn Gln Asn Thr
    130                 135                 140

Phe Glu Ser Met Ser Gly Leu Val Trp Thr Ser Ala Pro Ala Ser
145                 150                 155

<210>  22
<211>  159
<212>  PRT
<213>  Tobamovirus K1

<400>  22

Met Ser Tyr Pro Ile Thr Ser Pro Ser Gln Phe Val Phe Leu Ser Ser
1               5                   10                  15

Val Trp Ala Asp Pro Ile Glu Leu Leu Asn Val Cys Thr Asn Ser Leu
            20                  25                  30

Gly Asn Gln Phe Gln Thr Gln Gln Ala Arg Thr Thr Val Gln Gln Gln
        35                  40                  45

Phe Ser Glu Val Trp Glu Pro Phe Pro Gln Ser Thr Val Arg Phe Pro
    50                  55                  60

Gly Asp Val Tyr Lys Val Tyr Arg Tyr Asn Ala Val Leu Asp Pro Leu
65                  70                  75                  80

Ile Thr Ala Leu Leu Gly Thr Phe Asp Thr Arg Asn Arg Ile Ile Glu
                85                  90                  95

Val Glu Asn Arg Gln Ser Pro Thr Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Val Asn Glu Leu Val Arg Gly Thr Gly Leu Tyr Asn Gln Asn Thr
    130                 135                 140

Phe Glu Ser Met Ser Gly Leu Val Trp Thr Ser Ala Pro Ala Ser
145                 150                 155

<210>  23
<211>  159
<212>  PRT
<213>  Tobamovirus K2

<400>  23

Met Ser Tyr Pro Ile Thr Ser Pro Ser Gln Phe Val Phe Leu Ser Ser
1               5                   10                  15

Val Trp Ala Asp Pro Ile Glu Leu Leu Asn Val Cys Thr Asn Ser Leu
            20                  25                  30

Gly Asn Gln Phe Gln Thr Gln Gln Ala Arg Thr Thr Val Gln Lys Gln
        35                  40                  45

Phe Ser Glu Val Trp Lys Pro Phe Pro Gln Ser Thr Val Arg Phe Pro
    50                  55                  60

Gly Asp Val Tyr Lys Val Tyr Arg Tyr Asn Ala Val Leu Asp Pro Leu
65                  70                  75                  80

Ile Thr Ala Leu Leu Gly Thr Phe Asp Thr Arg Asn Ser Ile Ile Glu
                85                  90                  95

Val Glu Asn Arg Gln Ser Pro Thr Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Val Asn Glu Leu Val Arg Gly Thr Gly Leu Tyr Asn Gln Asn Thr
    130                 135                 140

Phe Glu Ser Met Ser Gly Leu Val Trp Thr Ser Ala Pro Ala Ser
145                 150                 155

<210>  24
<211>  159
<212>  PRT
<213>  Tobamovirus U2

<400>  24

Met Pro Tyr Thr Ile Asn Ser Pro Ser Gln Phe Val Tyr Leu Ser Ser
1               5                   10                  15

Ala Tyr Ala Asp Pro Val Gln Leu Ile Asn Leu Cys Thr Asn Ala Leu
            20                  25                  30

Gly Asn Gln Phe Gln Thr Gln Gln Ala Arg Thr Thr Val Gln Gln Gln
        35                  40                  45

Phe Ala Asp Ala Trp Lys Pro Val Pro Ser Met Thr Val Arg Phe Pro
    50                  55                  60

Ala Ser Asp Phe Tyr Val Tyr Arg Tyr Asn Ser Thr Leu Asp Pro Leu
65                  70                  75                  80

Ile Thr Ala Leu Leu Asn Ser Phe Asp Thr Arg Asn Arg Ile Ile Glu
                85                  90                  95

Val Asp Asn Gln Pro Ala Pro Asn Thr Thr Glu Ile Val Asn Ala Thr
            100                 105                 110

Gln Arg Val Asp Asp Ala Thr Val Ala Ile Arg Ala Ser Ile Asn Asn
        115                 120                 125

Leu Ala Asn Glu Leu Val Arg Gly Thr Gly Met Phe Asn Gln Ala Gly
    130                 135                 140

Phe Glu Thr Ala Ser Gly Leu Val Trp Thr Thr Thr Pro Ala Thr

145                 150                 155

<210>  25
<211>  159
<212>  PRT
<213>  Tobamovirus flavum

<400>  25

Met Ser Tyr Ser Ile Thr Thr Pro Ser Gln Phe Val Phe Leu Ser Ser
1               5                   10                  15

Ala Trp Ala Ala Pro Ile Glu Leu Ile Asn Leu Cys Thr Asn Ala Leu
            20                  25                  30

Gly Asn Gln Phe Gln Thr Gln Gln Ala Arg Thr Val Val Gln Arg Gln
        35                  40                  45

Phe Ser Glu Val Trp Lys Pro Ser Pro Gln Val Thr Val Arg Phe Pro
    50                  55                  60

Asp Ser Asp Phe Lys Val Tyr Arg Tyr Asn Ala Val Leu Asp Pro Leu
65                  70                  75                  80

Val Thr Ala Leu Leu Gly Ala Phe Asp Thr Arg Asn Arg Ile Ile Glu
                85                  90                  95

Val Glu Asn Gln Ala Asn Pro Thr Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Ile Val Glu Leu Ile Arg Gly Thr Gly Ser Tyr Asn Arg Ser Ser
    130                 135                 140

Phe Glu Ser Ser Ser Gly Leu Val Trp Thr Ser Gly Pro Ala Thr
145                 150                 155

<210>  26
<211>  158
<212>  PRT
<213>  Tobamovirus ORSV

<400>  26

Met Ser Tyr Thr Ile Thr Asp Pro Ser Lys Leu Ala Tyr Leu Ser Ser
1               5                   10                  15

Ala Trp Ala Asp Pro Asn Ser Leu Ile Asn Leu Cys Thr Asn Ser Leu
            20                  25                  30

Gly Asn Gln Phe Gln Thr Gln Gln Ala Arg Thr Thr Val Gln Gln Gln
        35                  40                  45

Phe Ala Asp Val Trp Gln Pro Val Pro Thr Leu Thr Ser Arg Phe Pro
    50                  55                  60

Ala Gly Ala Gly Tyr Phe Arg Val Tyr Arg Tyr Asp Pro Ile Leu Asp
65                  70                  75                  80

Pro Leu Ile Thr Phe Leu Met Gly Thr Phe Asp Thr Arg Asn Arg Ile
                85                  90                  95

Ile Glu Val Glu Asn Pro Gln Asn Pro Thr Thr Thr Glu Thr Leu Asp
            100                 105                 110

Ala Thr Arg Arg Val Asp Asp Ala Thr Val Ala Ile Arg Ser Ala Ile
        115                 120                 125

Asn Asn Leu Leu Asn Glu Leu Val Arg Gly Thr Gly Met Tyr Asn Gln
    130                 135                 140

Val Ser Phe Glu Thr Met Ser Gly Leu Thr Trp Thr Ser Ser
145                 150                 155

<210>  27
<211>  157
<212>  PRT
<213>  Tobamovirus PMMV

<400>  27

Met Ser Tyr Thr Ile Thr Asp Pro Ser Lys Leu Ala Tyr Leu Ser Ser
1               5                   10                  15

Ala Trp Ala Asp Pro Asn Ser Leu Ile Asn Leu Cys Thr Asn Ser Leu
            20                  25                  30

Gly Asn Gln Phe Gln Thr Gln Gln Ala Arg Thr Thr Val Gln Gln Gln
        35                  40                  45

Phe Ala Asp Val Trp Gln Pro Val Pro Thr Leu Thr Val Arg Phe Pro
    50                  55                  60

Ala Thr Gly Phe Lys Val Phe Arg Tyr Asn Ala Val Leu Asp Ser Leu
65                  70                  75                  80

Val Ser Ala Leu Leu Gly Ala Phe Asp Thr Arg Asn Arg Ile Ile Glu
                85                  90                  95

Val Glu Asn Pro Gln Asn Pro Thr Thr Ala Glu Thr Leu Asp Ala Thr
            100                 105                 110

Arg Arg Val Asp Asp Ala Thr Val Ala Ile Arg Ala Ser Ile Ser Asn
        115                 120                 125

Leu Met Asn Glu Leu Val Arg Gly Thr Gly Met Tyr Asn Gln Ala Leu
    130                 135                 140

Phe Glu Ser Ala Ser Gly Leu Thr Trp Ala Thr Thr Pro
145                 150                 155

<210>  28
<211>  159
<212>  PRT
<213>  Tobamovirus Rakkyo

<400>  28

Met Ser Tyr Asn Ile Asn Thr Pro Ser Gln Phe Val Phe Leu Ser Ser
1               5                   10                  15

Ala Trp Ala Asp Pro Ile Glu Leu Ile Asn Leu Cys Thr Asn Ala Leu
            20                  25                  30

Gly Asn Gln Phe Gln Thr Gln Gln Ala Arg Thr Val Val Gln Arg Gln
        35                  40                  45

Phe Ser Glu Val Trp Lys Pro Ser Pro Gln Val Thr Val Arg Phe Pro
    50                  55                  60

Asp Ser Asp Phe Lys Val Tyr Arg Phe Asn Ala Val Leu Asp Pro Leu
65                  70                  75                  80

Val Thr Ala Leu Leu Gly Ala Phe Asp Thr Arg Asn Arg Ile Ile Glu
                85                  90                  95

Val Glu Asn Gln Ala Asn Pro Ser Thr Ala Glu Thr Leu Asp Ala Thr
            100                 105                 110

Arg Arg Val Asp Asp Ala Thr Val Ala Ile Arg Ser Ala Ile Asn Asn
        115                 120                 125

Leu Ile Val Glu Leu Thr Arg Gly Thr Gly Ser Tyr Asn Arg Ser Ser
    130                 135                 140

Phe Glu Ser Ser Ser Gly Leu Val Trp Thr Ser Ser Pro Ala Thr
145                 150                 155

<210>  29

<211>  157
<212>  PRT
<213>  Tobamovirus RMV

<400>  29

Met Val Tyr Asn Ile Thr Ser Ser Asn Gln Tyr Gln Tyr Phe Ala Ala
1               5                   10                  15

Met Trp Ala Glu Pro Thr Ala Met Leu Asn Gln Cys Val Ser Ala Leu
            20                  25                  30

Ser Gln Ser Tyr Gln Thr Gln Ala Ala Arg Asp Thr Val Arg Gln Gln
        35                  40                  45

Phe Ser Asn Leu Leu Ser Ala Ile Val Thr Pro Asn Gln Arg Phe Pro
    50                  55                  60

Glu Thr Gly Tyr Arg Val Tyr Ile Asn Ser Ala Val Leu Lys Pro Leu
65                  70                  75                  80

Tyr Glu Ser Leu Met Lys Ser Phe Asp Thr Arg Asn Arg Ile Ile Glu
                85                  90                  95

Thr Glu Glu Glu Ser Arg Pro Ser Ala Ser Glu Val Ala Asn Ala Thr
            100                 105                 110

Gln Arg Val Asp Asp Ala Thr Val Ala Ile Arg Ser Gln Ile Gln Leu
        115                 120                 125

Leu Leu Asn Glu Leu Ser Asn Gly His Gly Leu Met Asn Arg Ala Glu
    130                 135                 140

Phe Glu Val Leu Leu Pro Trp Ala Thr Ala Pro Ala Thr
145                 150                 155


<210>  30
<211>  157
<212>  PRT
<213>  Tobamovirus crTMV

<400>  30

Met Ser Tyr Asn Ile Thr Asn Pro Asn Gln Tyr Gln Tyr Phe Ala Ala
1               5                   10                  15

Val Trp Ala Glu Pro Ile Pro Met Leu Asn Gln Cys Ile Ser Ala Leu
            20                  25                  30

Ser Gln Ser Tyr Gln Thr Gln Ala Ala Arg Asp Thr Val Arg Gln Gln
        35                  40                  45

Phe Ser Asn Leu Leu Ser Ala Val Val Ala Pro Ser Gln Arg Phe Pro
    50                  55                  60

Glu Thr Gly Ser Arg Val Tyr Val Asn Ser Ala Val Ile Lys Pro Leu
65                  70                  75                  80

Tyr Glu Ala Leu Met Lys Ser Phe Asp Thr Arg Asn Arg Ile Ile Glu
                85                  90                  95

Thr Glu Glu Glu Ser Arg Pro Ser Ala Ser Glu Val Arg Asn Ala Thr
            100                 105                 110

Gln Arg Val Asp Asp Ala Thr Val Ser Ile Arg Ser Gln Ile Gln Leu
        115                 120                 125

Leu Leu Ser Glu Leu Ser Ser Gly His Gly Tyr Met Asn Arg Ala Glu
    130                 135                 140

Phe Glu Ala Leu Val Pro Trp Thr Thr Ala Ala Ala Thr
145                 150                 155

<210>  31
<211>  157
<212>  PRT
<213>  Tobamovirus TVCV

<400>  31

Met Ser Tyr Asn Ile Thr Asn Pro Asn Gln Tyr Gln Tyr Phe Ala Ala
1               5                   10                  15

Val Trp Ala Glu Pro Ile Pro Met Leu Asn Gln Cys Met Ser Ala Leu
            20                  25                  30

Ser Gln Ser Tyr Gln Thr Gln Ala Ala Arg Asp Thr Val Arg Gln Gln
        35                  40                  45

Phe Ser Asn Leu Leu Ser Ala Val Val Thr Pro Ser Gln Arg Phe Pro
    50                  55                  60

Asp Thr Gly Ser Arg Val Tyr Val Asn Ser Ala Val Ile Lys Pro Leu
65                  70                  75                  80

Tyr Glu Ala Leu Met Lys Ser Phe Asp Thr Arg Asn Arg Ile Ile Glu
                85                  90                  95

Thr Glu Glu Glu Ser Arg Pro Ser Ala Ser Glu Val Ala Asn Ala Thr
            100                 105                 110

Gln Arg Val Asp Asp Ala Thr Val Ala Ile Arg Ser Gln Ile Gln Leu
        115                 120                 125

Leu Leu Ser Glu Leu Ser Asn Gly His Gly Tyr Met Asn Arg Ala Glu
    130                 135                 140

Phe Glu Ala Leu Leu Pro Trp Thr Thr Ala Pro Ala Thr
145                 150                 155

<210>  32
<211>  157
<212>  PRT
<213>  Tobamovirus TMV-cg

<400>  32

Met Val Tyr Asn Ile Thr Ser Ser Asn Gln Tyr Gln Tyr Phe Ala Ala
1               5                   10                  15

Met Trp Ala Glu Pro Thr Ala Met Leu Asn Gln Cys Val Ser Ala Leu
            20                  25                  30

Ser Gln Ser Tyr Gln Thr Gln Ala Ala Arg Asp Thr Val Arg Gln Gln
        35                  40                  45

Phe Ser Asn Leu Leu Ser Ala Ile Val Thr Pro Asn Gln Arg Phe Pro
    50                  55                  60

Glu Ala Gly Tyr Arg Val Tyr Ile Asn Ser Ala Val Leu Lys Pro Leu
65                  70                  75                  80

Tyr Glu Ser Leu Met Lys Ser Phe Asp Thr Arg Asn Arg Ile Ile Glu
                85                  90                  95

Thr Glu Glu Glu Ser Arg Pro Ser Ala Ser Glu Val Ala Asn Ala Thr
            100                 105                 110

Gln Arg Val Asp Asp Ala Thr Val Ala Ile Arg Ser Gln Ile Gln Leu
        115                 120                 125

Leu Leu Asn Glu Leu Ser Asn Gly His Gly Leu Met Asn Arg Ala Glu
    130                 135                 140

Phe Glu Val Leu Leu Pro Trp Ala Thr Ala Pro Ala Thr
145                 150                 155

<210>  33
<211>  157
<212>  PRT
<213>  Tobamovirus ORMV

<400>  33

Met Val Tyr Asn Ile Thr Ser Ser Asn Gln Tyr Gln Tyr Phe Ala Ala
1               5                   10                  15

Met Trp Ala Glu Pro Thr Ala Met Leu Asn Gln Cys Val Ser Ala Leu
            20                  25                  30

Ser Gln Ser Tyr Gln Thr Gln Ala Ala Arg Asp Thr Val Arg Gln Gln
        35                  40                  45

Phe Ser Asn Leu Leu Ser Ala Ile Val Thr Pro Asn Gln Arg Phe Pro
    50                  55                  60

Glu Ala Gly Tyr Arg Val Tyr Ile Asn Ser Ala Val Leu Lys Pro Leu
65                  70                  75                  80

Tyr Glu Ser Leu Met Lys Ser Phe Asp Thr Arg Asn Arg Ile Ile Glu
                85                  90                  95

Thr Glu Glu Glu Ser Arg Pro Ser Ala Ser Glu Val Ala Asn Ala Thr
            100                 105                 110

Gln Arg Val Asp Asp Ala Thr Val Ala Ile Arg Ser Gln Ile Gln Leu
        115                 120                 125

Leu Leu Asn Glu Leu Ser Asn Gly His Gly Leu Met Asn Arg Ala Glu
    130                 135                 140

Phe Glu Val Leu Leu Pro Trp Ala Thr Ala Pro Ala Thr
145                 150                 155


<210>  34
<211>  157
<212>  PRT
<213>  Tobamovirus CTMV-W

<400>  34

Met Ser Tyr Asn Ile Thr Asn Ser Asn Gln Tyr Gln Phe Phe Ala Ala
1               5                   10                  15

Val Trp Ala Glu Pro Ile Ala Met Leu Asn Gln Cys Ile Ser Ala Leu
            20                  25                  30

Ser Gln Ser Tyr Gln Thr Gln Ala Ala Arg Asp Thr Val Arg Gln Gln
        35                  40                  45

Phe Ser Asn Leu Leu Ser Ala Ile Val Thr Pro Asn Gln Arg Phe Pro
    50                  55                  60

Glu Thr Gly Tyr Arg Val Tyr Val Asn Ser Ala Val Leu Lys Pro Leu
65                  70                  75                  80

Tyr Glu Ala Leu Met Lys Ser Phe Asp Thr Arg Asn Arg Ile Ile Glu
                85                  90                  95

Thr Glu Glu Glu Ser Arg Pro Ser Ala Ser Glu Val Ala Asn Ala Thr
            100                 105                 110

Gln Arg Val Asp Asp Ala Thr Val Ala Ile Arg Ser Gln Ile Gln Leu
        115                 120                 125

Leu Leu Ser Glu Leu Ser Ser Gly His Gly Leu Met Asn Arg Ala Glu
    130                 135                 140

Phe Glu Val Leu Ile Pro Trp Ala Thr Ala Pro Ala Lys
145                 150                 155

<210>  35
<211>  493
<212>  PRT
<213>  Staphylococcus aureus

<400>  35

Ala Gln His Asp Glu Ala Gln Gln Asn Ala Phe Tyr Gln Val Leu Asn
1               5                   10                  15

Met Pro Asn Leu Asn Ala Asp Gln Arg Asn Gly Phe Ile Gln Ser Leu
            20                  25                  30

Lys Asp Asp Pro Ser Gln Ser Ala Asn Val Leu Gly Glu Ala Gln Lys
        35                  40                  45

Leu Asn Asp Ser Gln Ala Pro Lys Glu Ala Asp Ala Gln Gln Asn Asn
    50                  55                  60

Phe Asn Lys Asp Gln Gln Ser Ala Phe Tyr Glu Ile Leu Asn Met Pro
65                  70                  75                  80

Asn Leu Asn Glu Ala Gln Arg Asn Gly Phe Ile Gln Ser Leu Lys Asp
                85                  90                  95

Asp Pro Ser Gln Ser Thr Asn Val Leu Gly Glu Ala Lys Lys Leu Asn
            100                 105                 110

Glu Ser Gln Ala Pro Lys Asp Ala Asp Asn Asn Phe Asn Lys Glu Gln
        115                 120                 125

Gln Asn Ala Phe Tyr Glu Ile Leu Asn Met Pro Asn Leu Asn Glu Glu
    130                 135                 140

Gln Arg Asn Gly Phe Ile Gln Ser Leu Lys Asp Asp Pro Ser Gln Ser
145                 150                 155                 160

Ala Asn Leu Leu Ser Glu Ala Lys Lys Leu Asn Glu Ser Gln Ala Pro
                165                 170                 175

Lys Ala Ala Asp Asn Lys Phe Asn Lys Glu Gln Gln Asn Ala Phe Tyr
            180                 185                 190

Glu Ile Leu His Leu Pro Asn Leu Asn Glu Glu Gln Arg Asn Gly Phe
        195                 200                 205

Ile Gln Ser Leu Lys Asp Asp Pro Ser Gln Ser Ala Asn Leu Leu Ala
    210                 215                 220

Glu Ala Lys Lys Leu Asn Asp Ala Gln Ala Pro Lys Asx Ala Asp Asn
225                 230                 235                 240

Lys Phe Asn Lys Glu Gln Gln Asn Ala Phe Tyr Glu Ile Leu His Leu
                245                 250                 255

Pro Asn Leu Thr Glu Glu Gln Arg Asn Gly Phe Ile Gln Ser Leu Lys
            260                 265                 270

Asp Asp Pro Ser Val Ser Lys Glu Ile Leu Ala Glu Ala Lys Lys Leu
        275                 280                 285

Asn Asp Ala Gln Ala Pro Lys Cys Glu Glu Asp Asn Asn Lys Pro Gly
    290                 295                 300

Lys Glu Asp Asn Asn Lys Pro Gly Lys Glu Asp Asn Asn Lys Pro Gly
305                 310                 315                 320

Lys Glu Asp Asn Asn Lys Pro Gly Lys Glu Asp Asn Asn Lys Pro Gly
                325                 330                 335

Lys Glu Asp Asn Asn Lys Pro Gly Lys Glu Asp Gly Asn Lys Pro Gly
            340                 345                 350

Lys Glu Asp Asn Lys Lys Pro Gly Lys Glu Asp Gly Asn Lys Pro Gly
        355                 360                 365

Lys Glu Asp Asn Lys Lys Pro Gly Lys Glu Asp Gly Asn Lys Pro Gly
    370                 375                 380

Lys Glu Asp Gly Asn Lys Pro Gly Lys Glu Asp Gly Asn Gly Val His
385                 390                 395                 400

Val Val Lys Pro Gly Asp Thr Val Asn Asp Ile Ala Lys Ala Asn Gly
                405                 410                 415

Thr Thr Ala Asp Lys Ile Ala Ala Asp Asn Lys Leu Ala Asp Lys Asn
            420                 425                 430

Met Ile Lys Pro Gly Gln Glu Leu Val Val Asp Lys Lys Gln Pro Ala
        435                 440                 445

Asn His Ala Asp Ala Asn Lys Ala Gln Ala Leu Pro Glu Thr Gly Glu
    450                 455                 460

Glu Asn Pro Phe Ile Gly Thr Thr Val Phe Gly Gly Leu Ser Leu Ala
465                 470                 475                 480

Leu Gly Ala Ala Leu Leu Ala Gly Arg Arg Arg Glu Leu
                485                 490

<210>  36
<211>  128
<212>  PRT
<213>  Streptococcus

<400>  36

Ala Ala Glu Ala Gly Ile Thr Gly Thr Trp Tyr Asn Gln Leu Gly Ser
1               5                   10                  15

Thr Phe Ile Val Thr Ala Gly Ala Asp Gly Ala Leu Thr Gly Thr Tyr
            20                  25                  30

Val Thr Ala Arg Gly Asn Ala Glu Arg Arg Tyr Val Leu Thr Gly Arg
        35                  40                  45

Tyr Asp Ser Ala Pro Ala Thr Asp Gly Ser Gly Thr Ala Leu Gly Trp
    50                  55                  60

Thr Val Ala Trp Lys Asn Asn Tyr Arg Asn Ala His Ser Ala Thr Thr
65                  70                  75                  80

Trp Ser Gly Gln Tyr Val Gly Gly Ala Glu Ala Arg Ile Asn Thr Gln
                85                  90                  95

Trp Leu Leu Thr Ser Gly Thr Thr Glu Ala Asn Ala Trp Lys Ser Thr
            100                 105                 110

Leu Val Gly His Asp Thr Phe Thr Lys Val Lys Pro Ser Ala Ala Gly

        115                 120                 125

## Description

### FIELD OF THE INVENTION

The present invention relates to a process of affinity purifying a protein of interest using an affinity matrix comprising recombinant plant viral particles or recombinant plant virus-like particles. The invention further relates to the affinity matrix and to the recombinant viral particles, whereby the recombinant viral particles expose one or more recombinant proteins on their surface. The invention also relates to a fusion protein as a building block for said recombinant viral particles, to a polynucleotide encoding the fusion protein and to a plant, plant tissue or plant cells comprising said polynucleotide. The invention further relates to a process of producing said affinity matrix and to a process of producing said recombinant viral particles.

### BACKGROUND OF THE INVENTION

It is known that in microbial protein expression systems that have been optimized with regard to product yield, up to 90% of total production costs are the costs related to purification of the protein of interest from the host, rather than expenses for the production itself. In order to make protein production more economical, strategies are needed that will allow rapid and inexpensive separation of the protein of interest or non-proteinaceous small molecule of interest, from other contaminating components.

It has been proposed (WO02/068927) to use plant viral particles having short peptides (e.g. a FLAG-tag) bound to the surface of plant viral particles for purification of a protein of interest by affinity purification. However, in the process of the prior art, the protein of interest to be purified has to be fused to a protein such as a single chain antibody or other purification tag that is capable of binding to the peptide bound to the surface of the viral particles. It is therefore necessary to cleave the protein to be purified after the affinity purification step, which represents an additional process step that one would like to avoid.

Antibodies and antibody derivatives constitute about 20% of biopharmaceutical products currently in development. The purification of antibodies accounts for 50-80% of the total production costs (for review: Roque et al., 2004, Biotechnol. Prog., 20:639-654). Protein A from *Staphylococcus aureus* is widely used as an affinity protein in processes of immunoglobulin purification (for review: Jungbauer & Hahn, 2004, Curr. Opin. Drug. Disc. & Dev., 7:248-256).

Protein A reversibly interacts with the Fc domain of immunoglubulins (Lindmark et al., 1983, J. Immunol. Methods, 62:1-13; Gouda, et. al., 1998, Biochemistry, 37:129-136), predominantly via hydrophobic interactions (Dowd et al., 1998, Nat. Biotechnol., 16:190-195). The high stability and selectivity of protein A makes it a preferable generic ligand for immunoglobulin purification. The main source of protein A for the market has been recombinant protein A produced in *E. coli* (Duggleby & Jones, 1983, Nucleic Acids Res., 11:3065-3076; Engel et al., 1992, Protein Expr. Purif., 3:108-113). In prior art processes of purifying antibodies by affinity purification with protein A, protein A first has to be expressed and purified and then linked to a matrix such sepharose that is then used for affinity purification of the antibodies. Thus, the production of the affinity matrix involves many steps and is laborious and expensive. Due to the costs for the affinity matrix, the affinity matrix is typically used for several purification runs, leading to a risk of contamination between consecutive samples purified on the same affinity matrix. A cheaper and readily producible affinity matrix for the purification of antibodies is therefore much needed. Such a cheap affinity matrix could be a single used matrix, avoiding the contamination risk.

It is therefore an object of the invention to provide a process of affinity purifying a protein of interest, wherein no cleavage step has to be performed on the protein of interest after the affinity purification step. It is a further object of the invention to provide a process of purifying immunoglobulins such as therapeutic antibodies or fusion proteins thereof using an economical and easily accessible affinity matrix. It is another object of the invention to provide a process of purifying therapeutic antibodies without the risk of contamination with human or other animal pathogens. It is a further object of the invention to provide an affinity matrix for said purifying processes.

### SUMMARY OF THE INVENTION

The above objects are achieved by recombinant viral particles or recombinant plant virus-like particles comprising a plurality of fusion protein molecules, said fusion protein comprising the following fusion protein domains:
(i) a plant viral coat protein domain,
(ii) a recombinant protein domain, and
(iii) at least one peptide linker linking said plant viral coat protein and said recombinant protein,
wherein formation of said recombinant viral particle does not require free viral coat protein. Said recombinant domain or said coat protein domain may in turn have one or more than one domain.

The invention further provides recombinant viral particles or recombinant plant virus-like particles comprising fusion protein molecules, said fusion protein comprising the following fusion protein segments:
(i) a plant viral coat protein,
(ii) a recombinant protein comprising at least 50 amino acid residues and
(iii) a peptide linker linking said plant viral coat protein and said recombinant protein, said peptide linker comprising at least 10 amino acid residues,
wherein said viral particle comprises at most 20 mol% of free viral coat protein.

The invention further provides recombinant viral particles or recombinant plant virus-like particles comprising fusion protein molecules, said fusion protein comprising the following fusion protein segments:
(i) a plant viral coat protein of turnip vein clearing virus or a protein having an identity of at least 40 % to the coat protein of turnip vein clearing virus,
(ii) a recombinant protein comprising at least 50 amino acid residues and
(iii) a peptide linker linking said plant viral coat protein and said recombinant protein, said peptide linker comprising at least 10 amino acid residues.

The invention also provides a process of purifying a protein of interest, comprising the following steps:
(a) providing a plurality of said recombinant viral particles or recombinant virus-like particles comprising a fusion protein as defined above, said recombinant protein of said fusion protein having affinity to said protein of interest to be purified,
(b) contacting said affinity matrix with a liquid composition containing said protein of interest under conditions allowing binding of said protein of interest to said recombinant protein of said affinity matrix, whereby said affinity matrix is insoluble under said conditions,
(c) removing components of said liquid composition that have not bound to said recombinant protein from the mixture of step (b) under conditions preserving binding of said protein of interest to said recombinant protein of said affinity matrix, and
(d) separating said protein of interest from said affinity matrix. In this embodiment, said recombinant protein is also referred to as "affinity protein".

Said affinity matrix comprises the recombinant viral particles or the recombinant plant virus-like particles of the invention. Said affinity protein may have affinity to immunoglobulins or derivatives thereof, preferably said affinity protein is staphylococcal protein A or streptococcal protein G or a derivative thereof.

The invention further provides a process of producing recombinant plant viral particles or recombinant plant virus-like particles according to the invention, comprising expressing said fusion protein in a bacterium, in a plant, in plant tissue, or in plant cells. Further provided is a polynucleotide encoding the fusion protein defined above and a plant, plant tissue or plant cells comprising said polynucleotide.

Moreover, the invention provides recombinant viral material obtainable from the plant, the plant tissue or plant cells defined above.

Further, a fusion protein is provided comprising the following fusion protein segments: a plant viral coat protein, a linker peptide, and a staphylococcal protein A or a derivative of protein A capable of binding immunoglobulins, whereby said fusion protein is capable of forming viral particles or virus-like particles. Moreover, a fusion protein is provided comprising the following fusion protein segments: a plant viral coat protein, a linker peptide, and streptococcal protein G (alternatively: streptavidin or derivatives thereof) or a derivative of streptococcal protein G capable of binding to immunoglbulins.

Further, a kit-of-parts is provided comprising a plant, plant tissue or plant cells and a polynucleotide as defined above.

The invention also provides an affinity matrix for affinity purification of a protein of interest such as immunoglobulins, said affinity matrix comprising recombinant viral particles or recombinant plant virus-like particles comprising fusion protein molecules as defined above, wherein said recombinant viral particles comprise at most 20 mol% of free viral coat protein relative to the sum of free viral coat protein and said fusion protein present in said affinity matrix. "Free viral coat protein" is plant viral coat protein not fused to said recombinant protein of the invention.

The inventors of the present invention have found that immunoglobulins can be affinity purified using an affinity matrix comprising said recombinant viral particles or said recombinant plant virus-like particles having an affinity protein such as protein A or protein G bound to their surface. The invention is based on the finding of the inventors that it is possible to fuse a recombinant protein (such as protein A, protein G etc., or a derivative thereof) to plant viral coat protein without destroying the capability of the obtained fusion protein to assemble to viral particles. In one embodiment, the fusion protein is expressed in plant cells without co-expressing free viral coat protein. Viral particles assembled from said fusion protein have a high density of recombinant protein bound to the surface of said viral particles.

Before the present invention, there has not been a technology of producing plant viral particles comprising a recombinant protein on the surface of said particles as a fusion with plant viral coat protein, wherein the size of recombinant protein is not restricted to short peptides and wherein viral particle formation does not require providing viral coat protein in addition to the fusion protein of the viral coat protein and the recombinant protein. Here, the system of the invention is devoid of the limitations of the prior art: it does not require co-expression of free viral coat protein for the assembly of viral particles that display a recombinant protein on their surface. Consequently, the density of recombinant protein on the surface of the viral particles is much higher than in the case of viral particles comprising a high amount of free viral coat protein.

The fusion protein of the invention is a continuous polypeptide with an N-terminal end and a C-terminal end.

In one embodiment, the fusion protein comprises the following domains: a plant viral coat protein domain, a recombinant protein domain and at least one peptide linker linking said plant viral coat protein domain and said recombinant protein domain. In this embodiment, said recombinant protein may be present within the primary structure of the amino acid sequence of the coat protein, whereby the coat protein domain may be formed by two coat protein segments of the primary structure of the fusion protein. In this embodiment, said recombinant protein is linked to said coat protein by two peptide linkers. One peptide linker linking the N-terminal portion of said recombinant protein to the N-terminal segment of said coat protein, the second peptide linker linking the C-terminal portion of said recombinant protein to the C- terminal segment of said coat protein.

In another embodiment, the coat protein domain, the recombinant protein domain and one peptide linker are sequential segments in the primary structure of said fusion protein. In this embodiment, there are two possibilities for the sequence of the fusion protein segments from the N-terminus to the C- terminus of the fusion protein: (i) said plant viral coat protein is located at the N-terminus of the fusion protein and is followed by said peptide linker followed by said recombinant protein that is located at the C-terminal end of the fusion protein; (ii) said recombinant protein is located at the N-terminus of the fusion protein and is followed by said peptide linker, followed by said coat protein that is located at the C-terminal end of the fusion protein.

In all these embodiments, the fusion protein may comprise further amino acid residues or sequence segments at the N-terminus, at the C-terminus or within said fusion protein.

Said plant viral coat protein may be derived from any plant virus listed below. In one embodiment, said plant viral coat protein is derived from a plant virus forming rod-shaped viral particles. "Being derived" means that the coat protein used in the fusion protein of the invention does not have to be identical to the natural coat protein of a plant virus. Instead, the coat protein used in the fusion protein may have additions, deletions, insertions or mutations relative to a natural coat protein of a plant virus. It is only necessary that the coat protein maintains its capability to form viral or virus-like particles under suitable conditions. In one embodiment, at most 20 amino acid residues of the natural plant viral coat protein are deleted or mutated. In another embodiment, at most 20 amino acid residues are inserted into the natural sequence of the plant viral coat protein of the plant virus from which the coat protein of the invention is derived.

Said coat protein may be derived from a plus-sense single-stranded RNA virus. Examples of plant viruses the coat protein of which may be used in the present invention include tobamoviruses such as tobacco mosaic virus (TMV), turnip vein clearing virus, potato virus X, potato virus Y and fragments or homologues thereof, provided said fragments or homologues are capable of forming viral particles or virus-like particles under suitable conditions. Preferably, the coat protein of the invention has a sequence identity of at least 40% to the coat protein of turnip vein clearing virus, to tobacco mosaic virus, potato virus X or potato virus Y. In another embodiment, said sequence identity is at least 50%; in a further embodiment, said sequence identity is at least 60%. In an important embodiment, said coat protein has a sequence identity to the coat protein of tobacco mosaic virus of at least 80%.

The recombinant protein of the invention is exposed on the surface of said recombinant viral particles. There are no restrictions with regard to said recombinant protein. The type of said recombinant protein may be chosen depending on the application of the viral particles of the invention. The inventors have found for the first time that it is possible to create recombinant viral particles having a recombinant protein exposed on the surface of said viral particles without being restricted to small peptides of less than 40 or even less than 20 amino acids. Therefore, the invention shows its full potential with recombinant proteins having a size of at least 50 amino acid residues. However, in one embodiment, said recombinant protein has a size of at least 70 amino acid residues; in a further embodiment, said recombinant protein has a size of at least 90 amino acid residues; in a still further embodiment, said recombinant protein has a size of at least 110 amino acid residues.

The recombinant viral particles of the invention are plant viral particles in that the coat protein domain or segment of said fusion protein is derived from a plant virus. The viral particles of the invention are recombinant in that they are assembled from a coat protein that is part of the fusion protein of the invention. The recombinant viral particles of the invention are also referred to herein as "said viral particles".

Said recombinant protein may function as an affinity protein when a matrix of said viral particles is used for affinity purification of a protein of interest. Therefore, the terms "recombinant protein" and "affinity protein" are used interchangeably herein for a protein exposed on the surface of the viral particles of the invention. The recombinant protein of the invention is recombinant in that it is a segment or domain of the fusion protein of the invention.

For allowing affinity purification of a compound or protein of interest using the viral particles of the invention, said recombinant protein preferably has an affinity to the compound or protein of interest. In one embodiment, said recombinant protein has affinity to immunoglobulins or derivatives thereof such as therapeutic antibodies. In this case, said recombinant protein may be staphylococcal protein A or a domain or derivative thereof having affinity to immunoglobulins. In another embodiment, said recombinant protein may be streptococcal protein G or a derivative thereof capable of binding immunoglobulins. In another embodiment, said recombinant protein may be streptavidin or a derivative thereof such as strepactin having affinity to the StrepTagII.

If the compound to be purified is a small molecule, the recombinant protein can be any protein having affinity to said small molecule. For example, said recombinant protein can be an antibody or a single-chain fragment of an antibody having affinity to said small molecule.

The peptide linker of the invention links said plant viral coat protein and said recombinant protein in the primary structure of said fusion protein. The peptide linker allows assembly of viral particles of said fusion protein despite of the presence of said recombinant protein that may have a size of at least 50 amino acid residues. Said peptide linker should be flexible. In one embodiment, said peptide linker has no secondary structure in order to be flexible. In another embodiment, said peptide linker forms a helix. Preferably, said peptide linker does not form a β-sheet. It belongs to the general knowledge of the skilled person to design peptides having a predetermined secondary structure or no secondary structure. For example, proline residues break helices and β-sheets. One may therefore include one or more proline residues into said peptide linker. Alternatively, said peptide linker may contain a large proportion of glycine residues, whereby highly flexible peptide linkers may be obtained.

Said peptide linker preferably has at least 10 amino acid residues. In another embodiment, said peptide linker has at least 15 amino acid residues; in a further embodiment, said peptide linker has at least 20 amino acid residues; in a further embodiment, said peptide linker has at least 30 amino acid residues. The bigger the recombinant protein to be bound to the surface of said viral particle, the longer should the peptide linker be made. For example, if said recombinant protein has more than 200 amino acid residues, the peptide linker preferably has at least 25 amino acid residues.

The viral particles of the invention can be produced by expressing a polynucleotide encoding said fusion protein of the invention in a bacterial or plant host. Said plant host may be plant cells, plant tissue or entire plants. Apart from encoding said fusion protein, said polynucleotide will have regulatory elements required for the expression of said fusion protein in the chosen host. Upon expressing said polynucleotide, the viral particles of the invention generally assemble within host cells or may be assembled in vitro after isolating said fusion protein from the host cells under suitable conditions.

Said viral particles of the invention do not require the presence of free viral coat protein for assembly. Therefore, in one embodiment, said fusion protein is expressed without co-expressing free viral coat protein. The fusion protein of the invention can, however, assemble to said viral particles in the presence of free coat protein. In one embodiment, said viral particles comprise at most 30 mol-% free viral coat protein, preferably at most 20 mol-%, most preferably at most 10 mol-% of free viral coat protein. The content of free viral coat protein in said viral particles may be determined by solubilizing said viral particles and perform mass spectroscopy such as MALDI or ESI mass spectroscopy for determining the molecular weights and the relative abundance of the proteinaceous components of said viral particles. Any viral RNA contained in said viral particles may either be removed before performing mass spectroscopy or the signal thereof may be neglected when determining the relative abundance of the proteinaceous components of said viral particles.

In a further embodiment, an SDS-PAGE is performed on the viral particles and stained by Coomassie. The intensity of the band caused by free viral coat protein will be at most 20 %, preferably at most 10 % of the intensity of the band caused by said fusion protein, as determined by a commercial gel reader.

In one embodiment, the recombinant viral particles are produced in plant cells or plants using plant viral vectors, whereby the coat protein open reading frame (ORF) of a natural plant virus is replaced by the ORF of the fusion protein of the invention. The use of plant viral vectors has the advantage that high amounts of the fusion protein of the invention is produced per host cell, since the plant viral coat protein is the most abundant protein expressed in host cells after infection with a plant virus or plant viral vector. Further, cell to cell movement or systemic movement of the viral vector may lead to spread of the viral vector and to a high number of plant cells expressing said fusion protein. Methods of expressing a protein such as the fusion protein of the invention using a viral vector are known in the art. In one embodiment, the viral vector is introduced into plant cells or cells of a plant as part of a binary vector using Agrobacterium-mediated transformation.

The invention also provides an affinity matrix for purifying a compound or protein of interest. Said affinity matrix comprises a plurality of the viral particles of the invention. In one embodiment, said viral particles in said affinity matrix are not covalently cross-linked. In another embodiment, the viral particles in said affinity matrix may be cross-linked by a cross-linking agent. Cross-linking agents that can be used for cross-linking the viral particles of the invention are known in the art. Examples for such cross-linking agents are glutaraldehyde or bis-succinimids. A cross-linked affinity matrix has improved mechanical properties and a higher molecular weight. Further, covalent cross-linking allows to render said viral particles infection-deficient, which increases the safety of a product purified using said affinity matrix.

For purifying a protein of interest, the affinity matrix of the invention may be filled into a column for affinity chromatography. In another embodiment, said protein of interest may be purified using said affinity matrix by a batch method (cf. example 4). In any event, the affinity matrix of the invention is used in a solvent that does not dissolve said affinity matrix or said viral particles of the affinity matrix. Due to the high molecular weight and said insolubility of the affinity matrix, the affinity matrix can easily be separated (e.g. by sedimentation) from the soluble contaminants in a solution from which a protein of interest is to be purified.

A protein of interest to be purified according to the invention is typically present in dissolved form in an aqueous solution or dispersion further containing soluble or insoluble contaminants. An example of such a solution is a cell lysate. Insoluble matter is typically first separated by filtration or centrifugation for obtaining a clear solution. The clear solution may then be contacted with the affinity matrix of the invention, whereby the protein of interest binds to the affinity protein of said viral particles. Next, the affinity matrix having bound protein of interest is separated from the solution that originally contained the protein of interest. After washing the affinity matrix having bound protein of interest, the protein of interest may be detached from the affinity matrix under suitable conditions, whereby a solution containing purified protein of interest is obtained. A protocol for purifying immunoglobulins using an affinity matrix comprising viral particles having bound protein A is given in the examples.

### BRIEF DESCRIPTION OF THE DRAWINGS

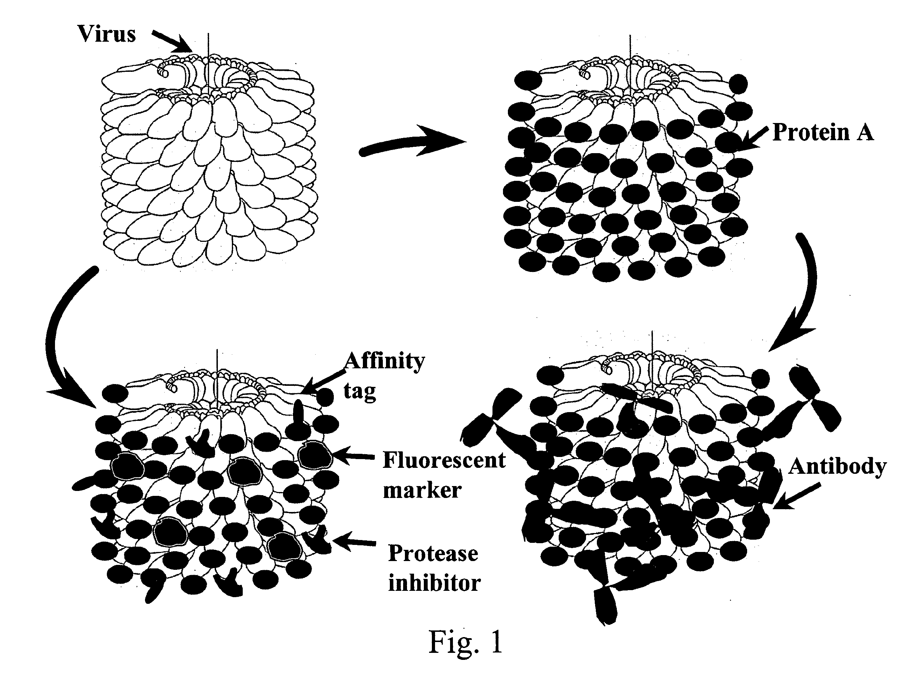

Figure 1 shows schematically at the top left side the structure of a plant viral particle. At the top right side, a viral particle according to the invention made up of the fusion protein of the invention is schematically shown, displaying protein A as said recombinant protein on the surface of the viral particle. At the bottom left, a viral particle according to the invention is shown and how of three different fusion proteins according to the invention, resulting in a viral particle displaying three different recombinant proteins on its surface, namely protein A, a fluorescent marker, and affinity tag. If the viral particle show at the top right side is used for affinity purification of the antibody, the viral particle binds antibody molecules via protein A.

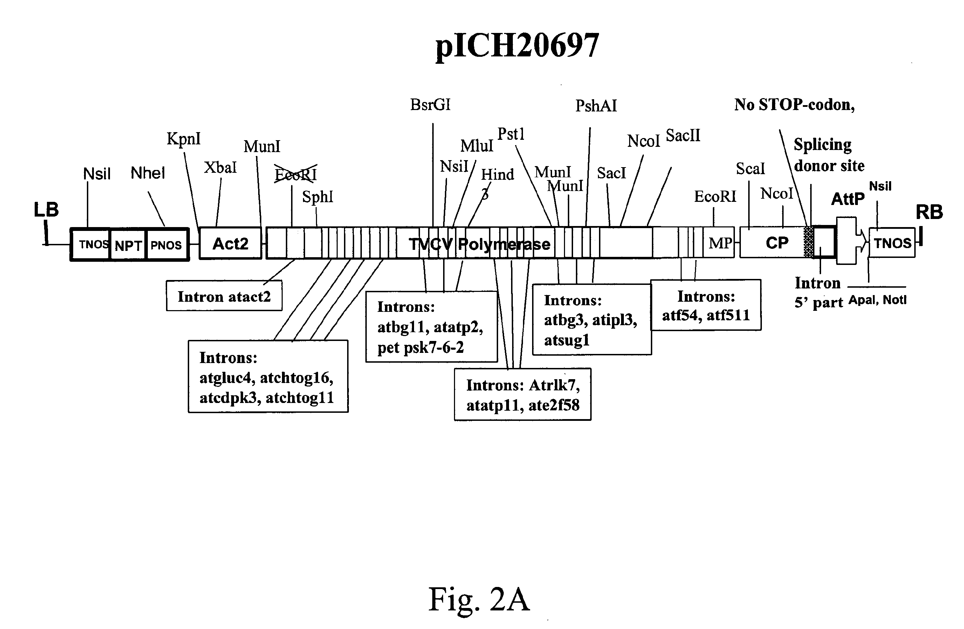

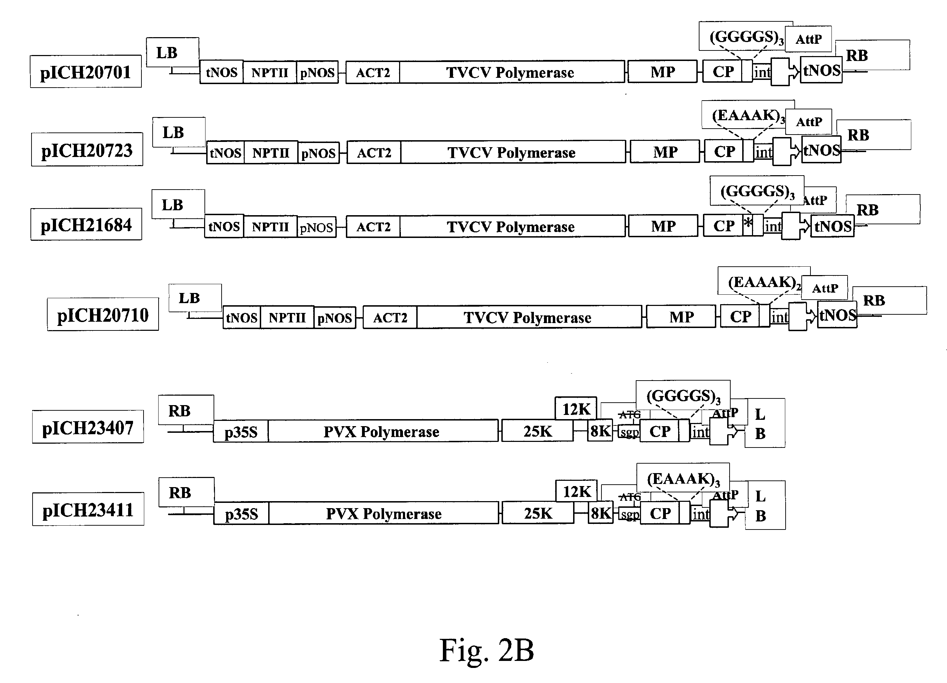

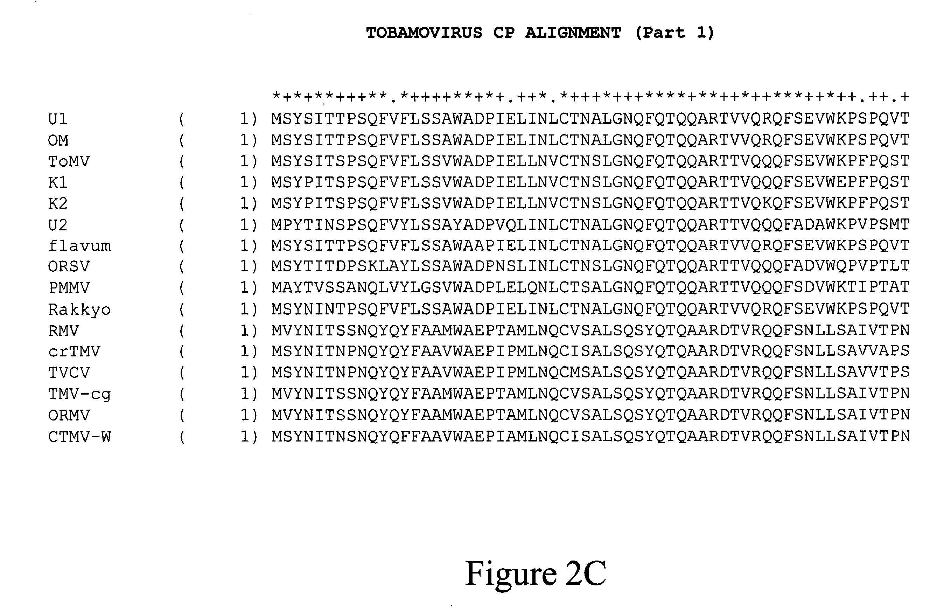

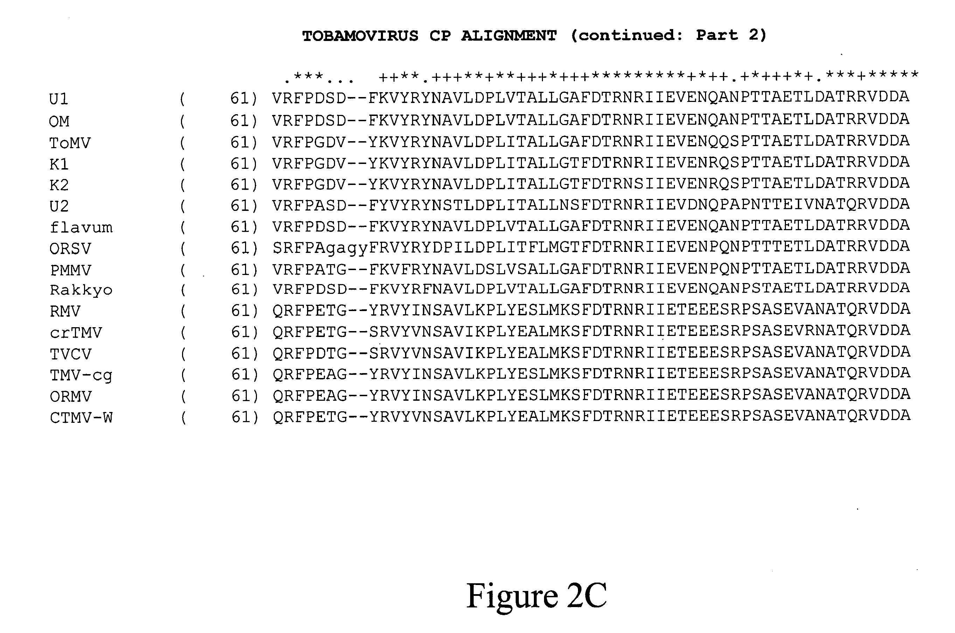

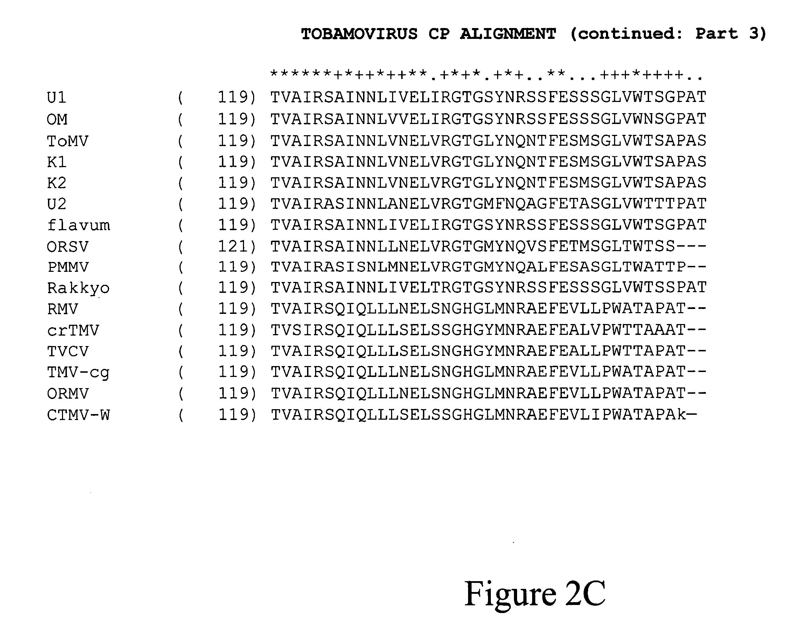

Figure 2 depicts T-DNA regions of different binary vectors:
(A) shows plCH20697;
(B) shows plCH20701, plCH20723, plCH21684, pICH20710, pICH23407 and plCH23411 containing 5' provectors of TMV-based and PVX-based viral expression systems (the intron positions are not shown but are identical to those of plCH20697 (Fig. 2A)).
   LB - left border of T-DNA; RB -right border of T-DNA; PNOS - promoter of agrobacterial nopaline synthase gene; TNOS - transcription termination region of agrobacterial nopaline synthase gene; NPTII - neomycin phoshotransferase II gene conferring resistance to kanamycin; P35S.- CaMV35S promoter; ACT2 - transcriptional enhancers of Arabidopsis actin2 gene promoter; MP - viral Movement protein; CP - viral coat protein; TVCV polymerase - RNA-dependent RNA polymerase of Turnip Vein Clearing Virus; PVX polymerase - RNA-dependent RNA polymerase of Potato Virus X; 25K, 12K, 8K - genes of triple gene block (TGB) region; sgp - subgenomic promoter; int
   - 5' part of intron sequence; AttP - recombination site recognised by site-specific integrase phiC31.
(C) - Alignment of CP amino acid sequences from different tobamoviruses performed with the GeneBee program (http://www.be)ozersky.msu.ru) (courtesy of Prof. Y. Dorokhov). The meaning of signs at the top of the alignment is as follows: '·' - the average weight of column pair exchanges is less than the weight matrix mean value; '.' - is less than mean value plus one SD; '+' - is less than mean value plus two SD; '*' - is more than mean value plus two SD. Gaps introduced for alignment are indicated by dashes. Data were taken from EMBL accession number J02415 for TMV U1 (U1), X00052 for TMV common strain (OM), X02144 for Tomato mosaic virus (ToMV), AJ243571 for TMV Kazakh strain (K1), Z92909 for TMV Kazakh strain (K2), M34077 for Tobacco green mottle virus or strain (U2), AF546184 for TMV flavum strain, AF321057 for Cucumber fruit mottle mosaic virus (CFMMV), D12505 for Cucumber green mottle mosaic virus (CGMMV), E04305 for Odontoglossum ringspot virus (ORSV), AJ308228 for Pepper mild mottle virus (PMMV), D63809 for TMV strain (Rakkyo), J02413 for sunnhemp mosaic virus (SHMV), AF254924 for ribgrass mosaic virus (RMV), Z29370 for crucifer strain of TMV (crTMV), U3387 for turnip vein-clearing virus (TVCV), U30944 for crucifer strain of TMV (TMV-Cg), AB003936 for crucifer strain of TMV Wasabi (CTMV-W), and Aguilar et al. (1996) for Oilseed rape mosaic virus (ORMV).

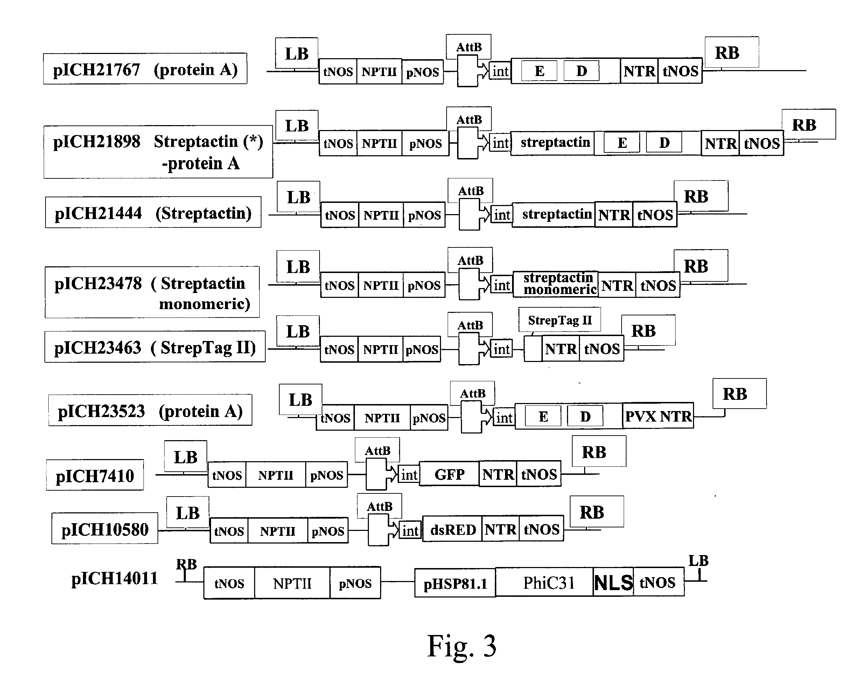

Figure 3 depicts T-DNA regions of binary vectors plCH21767, plCH21898, plCH21444, plCH323478, pICH23463, plCH23523, plCH7410, plCH10580 and pICH14011. LB - left border of T-DNA; RB -right border of T-DNA; PNOS - promoter of agrobacterial nopaline synthase gene; TNOS - transcription termination region of agrobacterial nopaline synthase gene; NPTII - neomycin phoshotransferase II gene conferring resistance to kanamycin; NTR - 3' non-translated region of viral RNA; AttB - recombination site recognised by site-specific integrase phiC31; pHSP81.1 promoter of gene encoding for Arabidopsis heat shock protein hsp81.1; NLS - nuclear localization signal; GFP - gene encoding synthetic green fluorescent protein; dsRED - red fluorescent protein; E, D - immunoglobulin binding domains E and D of *Staphylococcus aureus* protein A.

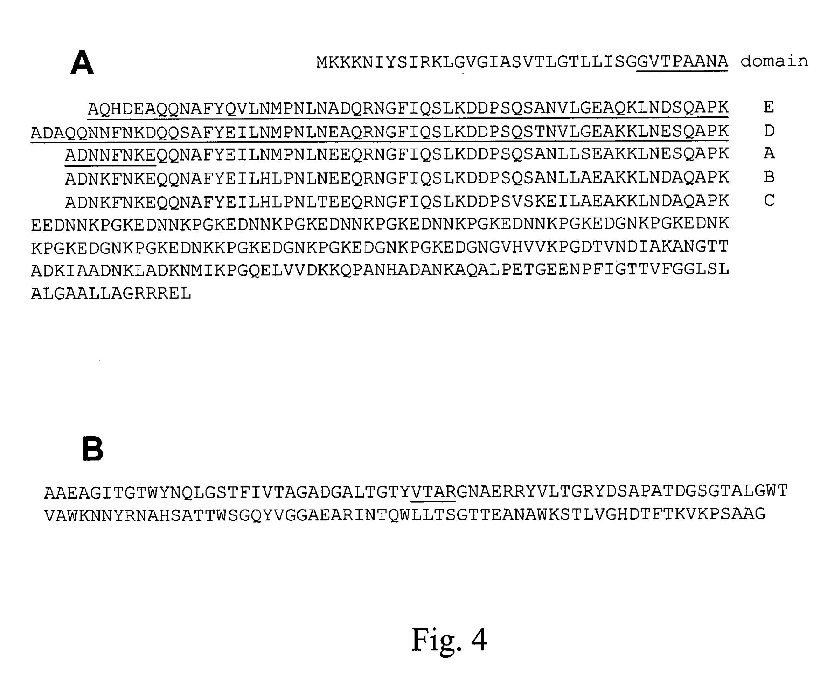

Figure 4 shows the sequences of (A) *Staphylococcus aureus* protein A and (B) a fragment of mature streptavidin (amino acid residues 12 - 139).

The part of protein A gene encoding for the underlined region of the protein sequence was re-synthesized with a codon usage optimized for expression in *N. tabacum* and for the structure and stability of the mRNA. It was cloned into TMV-3' provector (pICH21767).

The length of the cloned sequence is 133 aa (domain E: 56 aa; domain D: 61 aa).

The sequence fragment of mature streptavidin (aa 12-139) was cloned into the 3'-provector pICH21444 for fusion to CP; the mutated residues for increased affinity to StrepTagll are underlined (native sequence at this position is "ESAV").

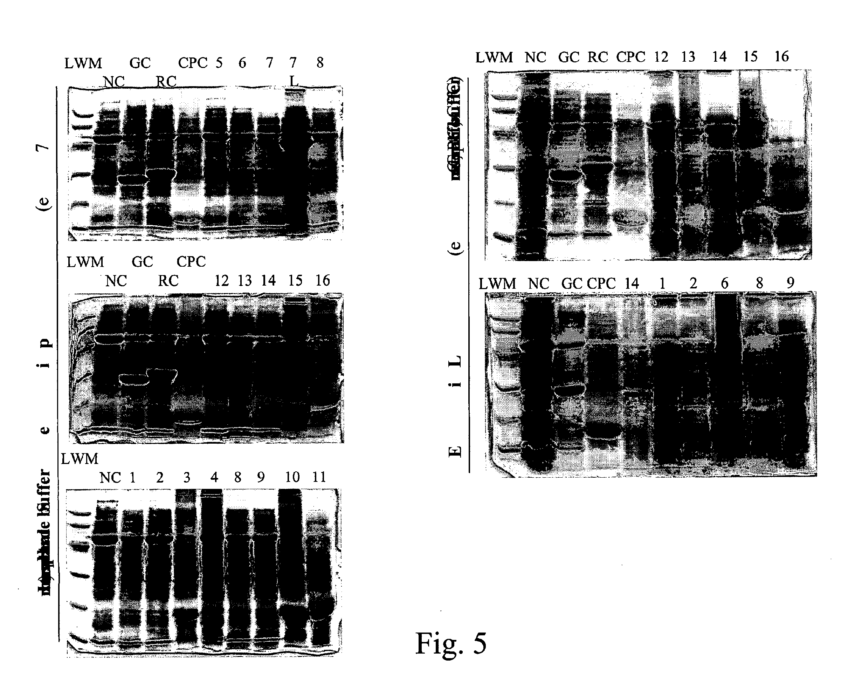

Figure 5 shows electrophoretic analysis of different fusions of recombinant protein with CP. Gels on the left: Standard extracts in phosphate buffer (except 7L). Gels on the right: Extracts in Laemli buffer (except for GC, RC, CPC)
1) pICH20697-pICH7410 (GFP);
2) pICH20697-pICH10580 (dsRED);
3) pICH20697-pICH7410 (GFP), systemic leaves;
4) 20697- plCH10580 (dsRED), systemic leaves;
5) plCH20701;
6) pICH20701- plCH7410 (GFP);
7) pICH20701- pICH10580 (dsRED);
8) pICH20710- plCH7410 (GFP);
9) pICH20710- plCH10580 (dsRED;
10) pICH20710- plCH7410 (GFP), systemic leaves;
11) pICH20710- pICH10580 (dsRED), systemic leaves;
12) pICH20723;
13) pICH20723-pICH7410 (GFP);
14) pICH20723- pICH10580 (dsRED);
15) pICH20723- pICH7410 (GFP), systemic leaves;
16) pICH20723- pICH10580 (dsRED), systemic leaves.
   NC - negative control; GC - GFP control; RC - dsRED control; CPC - CP control; L - Laemli buffer.

Figure 6
(A) - Electron microscopy of recombinant viral particles. The bars represent 100 nm.
(B)- Electrophoretic separation of protein extracted from inoculated leaf tissue.
   1) pICH20701-pICH21767 (protein-A, 2 domains);
   2) pICH20701-pICH21770 (protein-A, 1 domain);
   3) pICH20723-pICH21767 (2 domains);
   4) pICH20723-pICH21770 (1 domain);
   5) wild-type virus.
(C)- Electrophoretic separation of protein extracted from recombinant viral particles.
   1) wild-type virus;
   2 - 7) plCH20701-plCH21767 (protein A, 2 domains), different preparations of viral particles;
   8) MW protein markers.

Figure 7. Measurement of antibody binding capacity of protein A displayed on the surface of plant virus-derived matrix. s - supernatant; p - pellet; HC - heavy chain of IgG; LC - light chain of IgG; CP-protA - viral coat protein-protein A fusion; MW - molecular weight protein markers (kDa).

Figure 8. Purification of IgG from plant extracts using viral particles displaying protein A on their surface.
1) Crude extract;
2) Supernatant after precipitation of IgG;
3) Resuspended pellet after precipitation of IgG;
4) Resuspended pellet after removal of particles;
5) Resuspended pellet after PEG-precipitation of IgG (2x concentrated compared to other samples).
   HC - heavy chain of IgG; LC - light chain of IgG; CP-protA - viral coat protein-protein A fusion; MW - molecular weight protein markers (kDa).

### DETAILED DESCRIPTION OF THE INVENTION

Viral coat protein is the main building block of viral particles and virus-like particles (VLP). Viral particles and VLP are structured multimolecular biopolymers. By fusing a recombinant protein with a viral coat protein, it is possible to obtain viral particles with foreign epitopes on their surface. The translational fusion of a recombinant protein with a viral coat protein (Hamamoto et al., 1993, BioTechnology, 11, 930-932; Gopinath et al., 2000, Virology, 267, 159-173; Porta et al., 1994, Virology, 202:949-955; Porta et al., 2003, Virology, 310: 50-63; JP6169789; US5977438; WO02068927) has, however, been restricted in the prior art, as the recombinant protein that could be fused to a plant viral coat protein without affecting viral particle assembly has been limited to 20-25 amino acid residues (Turpen et al., 1995 Biotechnology, 13: 53-57; Sugiyama et al., 1995, FEBS Lett., 359: 247-250). Therefore, in the prior art, significant amounts (up to 95%) of free viral coat protein have been co-expressed with the fusion protein for co-assembly with the fusion protein, whereby viral particles consisting of up to 95% of free vial coat protein are obtained. Obviously, this limits any application of viral particles displaying a peptide on the surface.

In general, the limited size of peptides that could be fused to the CPs of plant viruses such as TMV, cowpea mosaic virus (CPMV), alfalfa mosaic virus, etc., while retaining the ability to assemble into functional virions has restricted, in the prior art, these systems to the expression of short immunogen epitopes and peptide hormones. Another publication (Santa-Cruz et al., 1996, Proc Natl Acad Sci U S A, 93:6286-6290) describes formation of Potato Virus X (PVX) virions containing on its surface green fluorescent protein (GFP) expressed as N-terminal fusion with potato virus X coat protein. However, as in the cases with TMV vectors, the expression of significant amount of wild type coat protein was necessary for assembly of viral particles. The latter was achieved either by expression of viral coat protein from independent vector stably integrated into plant chromosomal DNA or by using a cleavable linker peptide in GFP-CP fusion, thus providing a source for CP for virion formation. It was not possible to obtain viral particles displaying GFP on their surface in the absence of free viral coat protein. It was shown in another work (Bendahmane et al., 2002, Proc. Natl. Acad. Sci. USA, 99:3645-3650) that fusion of GFP with coat protein of tobacco mosaic virus in the absence of a peptide linker did not produce recombinant viral particles.

There are many applications for which the recombinant viral particles of the invention are useful such as affinity chromatography. For example, purification of antibodies and antibody derivatives that constitute 20% of biopharmaceutical products currently in development, accounts for 50-80% of total manufacturing costs (for review: Roque et al., 2004, Biotechnol. Prog., 20:639-654). Protein A from *Staphylococcus aureus* is broadly used as affinity protein in the process of immunoglobulin purification (for review: Jungbauer & Hahn, 2004, Curr. Opin. Drug. Disc. & Dev., 7:248-256). Protein A reversibly interacts with the Fc domain of immunoglubulins (Lindmark et al., 1983, J. Immunol. Methods, 62:1-13; Gouda, et. al., 1998, Biochemistry, 37:129-136), predominantly via hydrophobic interactions (Dowd et al., .1998, Nat. Biotechnol., 16:190-195). The high stability and selectivity of protein A makes it a useful generic affinity protein for immunoglobulin purification. The main source of protein A for the market has been recombinant protein A produced in *E. coli* (Duggleby & Jones, 1983, Nucleic Acids Res., 11:3065-3076; Engel et al., 1992, Protein Expr. Purif., 3:108-113). Display of protein A as affinity protein on the surface of an affinity matrix, such as matrix comprising viral particles or virus-like particles opens the opportunity for a cheap source of an affinity matrix having bound protein A or having bound another immunoadsorbent to be used in downstream processing of recombinant monoclonal antibodies. Another protein that can be used in this invention is streptococcal protein G (Guss et al., 1986, EMBO J., 5: 1567-1575), that also has strong affinity to Fc domain of IgG (Sauer-Eriksson et al., 1995, Structure, 3:275-278) and also weak affinity to the Fab fragment (Derrick & Wigley, 1992, Nature, 359: 752-754).

The present invention utilizes various properties of plant viruses for the purposes of purifying and visualizing proteins of interest produced in different hosts (which for purposes of this invention is meant to include any biological protein production host or any non-biological protein production method). The general principle of the invention is shown in Figure 1: plant viral particles displaying one or more recombinant protein(s) on their surface and the use of said plant viral particles for the purification of a protein of interest (antibodies). Also, the present invention utilizes the ability of viral coat protein to polymerize and form highly organized protein structures. The definition "viral particle" of the invention covers plant viral particles and plant virus-like particles (VLP) that contain a fusion protein comprising viral coat and a recombinant protein of interest in accordance with the claims of this invention. The terms "protein matrix" or "affinity matrix" mean a plurality of plant viral particles that together form a matrix comprising viral particles according to the invention. A rod-shaped viral particle is schematically shown in Figure 1. However, practically any sufficiently characterized plant virus can be adopted for practicing this invention. DNA and RNA viruses belonging to different taxonomic groups are suitable for constructing fusion protein comprising a plant viral coat protein.

A list of viruses to which this invention can be applied is presented below. Taxa names in quotes (and not in italic script) indicate that this taxon does not have an ICTV international approved name. Species (vernacular) names are given in regular script. Viruses with no formal assignment to genus or family are indicated):
**DNA Viruses: Circular dsDNA Viruses:** Family: *Caulimoviridae,* Genus: *Badnavirus, **type species:*** commelina yellow mottle virus, Genus: *Caulimovirus, **Type species:*** cauliflower mosaic virus, Genus "SbCMV-like viruses", ***Type species:*** Soybean chloroticmottle virus, Genus "CsVMV-like viruses", ***Type species:*** Cassaya vein mosaicvirus, Genus "RTBV-like viruses", ***Type species:*** Rice tungro bacilliformvirus, Genus: "Petunia vein clearing-like viruses", ***Type species:*** Petunia vein clearing virus; **Circular ssDNA Viruses:** Family: *Geminiviridae,* Genus: Mastrevirus (Subgroup I Geminivirus), ***Type species:*** maize streak virus. Genus: *Curtovirus* (Subaroup II Geminivirus), ***Type species:*** beet curly top virus, Genus: *Begomovirus* (Subaroup III Geminivirus). ***Type species:*** bean golden mosaic virus;
**RNA Viruses:**
   **ssRNA Viruses:** Family: *Bromoviridae,* Genus: *Alfamovirus, **Type species:*** alfalfa mosaic virus, Genus: *Ilarvirus, **Type species:*** tobacco streak virus, Genus: *Bromovirus, **Type species:*** brome mosaic virus, Genus: Cucumovirus, **Type species:** cucumber mosaic virus;
   Family: *Closteroviridae,* Genus: *Closterovirus, **Type species:*** beet yellows virus, Genus: *Crinivirus, **Type species:*** Lettuce infectious yellows virus, Family: *Comoviridae,* Genus: *Comovirus, **Type species:** cowpea mosaic virus,* Genus: *Fabavirus, **Type species:*** broad bean wilt virus 1, Genus: *Nepovirus, **Type species:*** tobacco ringspot virus;
   Family: *Potyviridae,* Genus: *Potyvirus, **Type species:*** potato virus Y, Genus: *Rymovirus, **Type species:** ryegrass mosaic virus,* Genus: *Bymovirus, **Type species:*** barley yellow mosaic virus;
   Family: *Sequiviridae,* Genus: *Sequivirus, **Type species:*** parsnip yellow fleck virus, Genus: *Waikavirus, **Type species:*** rice tungro spherical virus; Family: *Tombusviridae,* Genus: *Carmovirus, **Type species:*** carnation mottle virus, Genus: *Dianthovirus, **Type species:*** carnation ringspot virus, Genus: *Machlomovirus, **Type*** species: maize chlorotic mottle virus, Genus: *Necrovirus, **Type species:*** tobacco necrosis virus, Genus: *Tombusvirus, Type species:* tomato bushy stunt virus, **Unassigned Genera of ssRNA viruses,** Genus: *Capillovirus, **Type species:*** apple stem grooving virus;
   Genus: *Carlavirus, **Type species:*** carnation latent virus; Genus: *Enamovirus, **Type species:*** pea enation mosaic virus,
   Genus: *Furovirus, **Type species:*** soil-borne wheat mosaic virus, Genus: *Hordeivirus, **Type species:*** barley stripe mosaic virus, Genus: *Idaeovirus, **Type species:*** raspberry bushy dwarf virus;
   Genus: *Luteovirus, **Type species:*** barley yellow dwarf virus; **Genus:** *Marafivirus, **Type species:*** maize rayado fino virus; **Genus:** *Potexvirus, **Type species:*** potato virus X; Genus: *Sobemovirus, **Type species:*** Southern bean mosaic virus, Genus:
      *Tenuivirus, **Type species:*** rice stripe virus,
      Genus: *Tobamovirus, **Type species:*** tobacco mosaic virus,
      Genus: *Tobravirus, **Type species:*** tobacco rattle virus,
      Genus: *Trichovirus, **Type species:*** apple chlorotic leaf spot virus; Genus: *Tymovirus, **Type species:*** turnip yellow mosaic virus; Genus: *Umbravirus, **Type species:*** carrot mottle virus;
   **Negative ssRNA Viruses:** Order: *Mononegavirales,* Family: *Rhabdoviridae,* Genus: *Cytorhabdovirus, **Type Species***: lettuce necrotic yellows virus, Genus: *Nucleorhabdovirus,* ***Type* species:** potato yellow dwarf virus;
   **Negative ssRNA Viruses:** Family: *Bunyaviridae,* Genus: *Tospovirus,* ***Type species*:** tomato spotted wilt virus;
   **dsRNA Viruses:** Family: *Partitiviridae,* Genus: *Alphacryptovirus,* ***Type species*:** white clover cryptic virus 1, Genus: *Betacryptovirus,* ***Type species*:** white clover cryptic virus 2, Family: *Reoviridae,* Genus: *Fijivirus, **Type species:*** Fiji disease virus, Genus: *Phytoreovirus, **Type species***: wound tumor virus, Genus: *Oryzavirus,* ***Type species*:** rice ragged stunt virus;
   **Unassigned Viruses:**
      Genome: *ssRNA,* **Species** Garlic viruses A,B,C,D, **Species** grapevine fleck virus, **Species** maize white line mosaic virus, **Species** olive latent virus 2, **Species:** ourmia melon virus, **Species** Pelargonium zonate spot virus.

The viruses (tobacco mosaic virus and potato virus X) used in the examples were predominantly chosen because of the ready availability of well-established expression systems for said viruses (Donson et al., 1991, Proc Natl Acad Sci U S A, 88:7204-7208; Shivprasad et al., 1999, Virology, 255:312-323; Marillonnet et al., 2004, Proc Natl Acad Sci U S A, 101:6852-6857; Marillonnet et al., 2005, Nat Biotechnol., 23:718-723; Chapman, Kavanagh & Baulcombe, 1992, Plant J., 2:549-557; Baulcombe, Chapman & Santa Cruz, 1995, Plant J., 7:1045-1053; Angell & Baulcombe, 1997, EMBO J., 16:3675-3684) including the very recently developed system for expression of hetero-oligomeric proteins (EP Application No. 05 001 819.1). Other plant viruses including DNA viruses also can be used for practicing this invention (for reviews please refer to: Mullineaux et al., 1992, Genetic Engineering in Plant Viruses, CRC Press Inc., pp187-215; Timmermans et al., 1994, Ann. Rev. Plant Physiol. Plant Mol. Biol., 45:79-112; Porta & Lomonossoff, 2002, Biotechnol. Genet. Engineering Rev., 19:245-291).

We have surprisingly found that (a) flexible peptide linker(s) (either unable to form secondary structure or capable of forming a helical secondary structure) allows overcoming size restrictions in generating translational fusion of recombinant protein with plant viral coat protein. Said linker peptide removes or significantly reduces a negative effect of fusion partners on each other's functionality. The linker peptides used in this invention may be flexible peptide linkers such as (GGGGS)ₙ or helix-forming peptide linkers such as (EAAAK)ₙ, wherein n may be 2 - 5. The peptide linkers are segments of said fusion protein. The use of this type of peptide linkers in fusion proteins of different proteins and their effect on the function of fusion proteins is described (Arai et al., 2001, Protein Eng., 14:529-532; Arai et al., 2004, Proteins, 57:829-838). In example 1 we describe the design of constructs containing linker peptides with n=3 (see also Fig. 1). However, similar constructs with n=2 (not shown) were also made. We found that longer peptide linkers (n=3) have significant positive impact on the function of fusion partners: viral CP (to form viral particle) and recombinant protein (for example, protein A to bind immunoglobulins). We assume that longer linkers (longer than 15 amino acid residues) would further minimize the interference between the functions of the fusion partners. However, the length of linker peptides suitable for practicing this invention can be significantly longer than mentioned above (up to 25 amino acid residues) and might depend on the choice of recombinant protein to be fused to viral coat protein. The choice of the peptide linker for practicing this invention is not limited to the linkers described above. Many other types of linkers can be used in this invention. Typically, the linkers in multi-domain or multi-repeat proteins have little secondary structure, but there are other types of linkers that form helical structures (Ortiz et al., 2005, J. Mol. Biol., 349:638-647). Also, there is abundant information about linkers design in phage display technology (Maruyama et al., 1994, Proc. Natl. Acad. Sci. USA, 91:8273-8277; Turner et al., 1997, J. Immunol. Methods, 205:43-54; Castillo et al., 2001, J. Immunol. Methods, 257:117-122; Weiss et al., 2000, Protein Sci., 9:647-654; Mikawa et al., 1996, J. Mol. Biol., 262:21-30). Despite significant difference in structure and biology between phage and plant virus-based systems, general principle for the choice of linker can be applied for plant viral vectors. It appears that distancing the recombinant protein from the viral coat protein via said peptide linker peptide reduces interaction between the coat protein and the recombinant protein on the fusion protein and, as a consequence, preserves their functions. The peptide linker chosen for practicing this invention can be tested for its suitability in this application using different programs predicting protein secondary structures (e.g. NNPREDICT, link hftp://www.cmpharm.ucsf.edu/-nomi/nnpredict.html) (Kneller, et al., 1990, J. Mol. Biol. 214: 171-182). For predicting secondary structure, the linker peptide shall be analyzed as integrated part of the fusion protein due to possible influence of flanking sequences (coat protein and recombinant protein of interest) on its secondary structure. Alternative program can be PredictProtein at Heidelberg University, Germany (http://www.embt-heidetberg.de/predictprotein/predictprotein.htmt).

The recombinant plant viral particles or plant virus-like particles can be produced by expressing said fusion protein of the invention. In one embodiment, said fusion protein is expressed in plant cells or plants using plant viral vectors. In such plant viral vectors, the coat protein of the virus from which the viral vector is derived is replaced by a polynucleotide encoding said fusion protein of the invention.

Plant viral vectors are efficient tools for transient high yield expression of recombinant proteins such as the fusion protein of the invention in plants (for review see: Porta & Lomonossoff, 1996, Mol. Biotechnol., 5, 209-221; Yusibov et al., 1999, Curr. Top. Microbiol. Immunol., 240, 81-94; Gleba et al., 2004, Curr Opin Plant Biol. 7:182-188; Gleba et al., 2005, Vaccine, 23:2042-2048). Viral vector-based expression systems offer a significantly higher yield of transgene product (such as the fusion protein of the invention) compared to plant nuclear transgenes. For example, the level of recombinant protein can reach 5-50% of the total cellular plant protein content, when expressed from a viral vector (Kumagai et al., 2000, Gene, 245, 169-174; Shivprasad et al., 1999, Virology, 255, 312-323; Marillonnet et al., 2004, Proc Natl Acad Sci U S A, 101:6852-6857; Marillonnet et al., 2005, Nat Biotechnol., 23:718-723). There are several published patents which describe viral vectors suitable for systemic expression of the fusion protein of the invention in plants (US5316931; US5589367; US5866785). In general, these vectors can express a foreign gene as a translational fusion with a viral protein (US5491076; US5977438), from an additional subgenomic promoter (US5466788; US5670353; US5866785), or from polycistronic viral RNA using IRES elements for independent protein translation (WO0229068). Other systems (WO2005049839) rely on agrobacteria for systemic delivery of viral replicon and do have significantly higher capacity for the size of a foreign gene compared to systemic viral vectors.

In example 2 of present invention we describe the production and analysis of viral particles displaying different recombinant proteins of different size on its surface. The electrophoretic analysis of different viral CP-recombinant protein fusions expressed with the help of a viral vector is shown in Fig. 5. It is evident from the results of said analysis that the majority of tested fusions showed a high expression level in infiltrated leaves and in some cases, recombinant viral vectors could move systemically. However, systemic movement quite frequently leads to the reversion to wild type vector. Therefore, in one embodiment of the invention, the CP-recombinant protein fusions are expressed in inoculated leaves using agrobacterium-mediated delivery of viral vectors or provectors (Marillonnet et al., 2004, Proc Natl Acad Sci U S A, 101:6852-6857; Marillonnet et al., 2005, Nat Biotechnol., 23:718-723). Out of seven different recombinant proteins expressed as fusions with CP according to the invention, six recombinant proteins were successfully expressed and isolated from the plant tissue in form of protein matrix. The activity of the recombinant proteins displayed on the surface of said viral particles was confirmed experimentally. It is also evident that no expression of recombinant proteins fused to CP directly, without linker peptide, was observed neither in inoculated nor in systemic leaves (lanes 1, 2, 3, 4, Fig. 5). High level of expression in inoculated leaves was achieved with the same recombinant proteins fused to CP via said peptide linker of the invention (lanes 7L, 13, 14; Fig. 5).

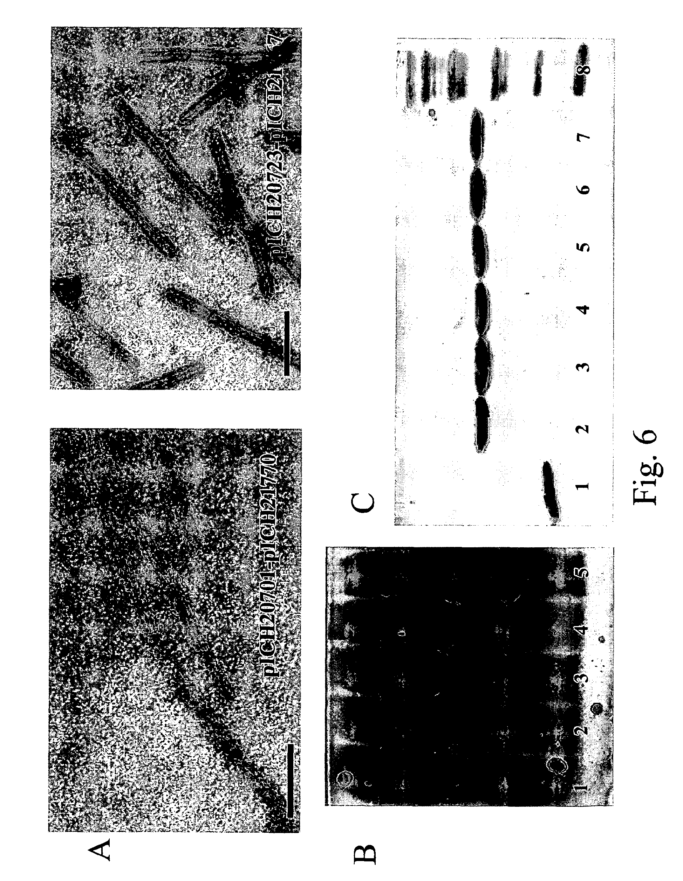

Another embodiment of this invention demonstrates the functionality of a recombinant protein displayed on the surface of a viral particle in biotechnology applications. We have chosen fusion proteins comprising the domains E and D of protein A (133 amino acid residues, see Fig. 4-A) with viral CP via a 15 amino acid peptide linker. Protein A is an efficient affinity tag broadly used in chromatographic purification of immunoglobulins, preferentially IgG and their functional derivatives (Fuglistaller, P., 1989, J. Immunol. Methods, 124-171-177; Fahmer et al., 1999, Biotechnol. Appl. Biochem., 30:121-128; Jungbauer & Hahn, 2004, Curr. Opin. Drug Discov. Dev., 7:248-256). There is a growing demand for recombinant protein A to be used in purification of recombinant immunoglobulins, as the number of monoclonal antibodies in clinical trials is steadily growing and for commercial production of said antibodies large quantities of recombinant protein A will be required. Additionally, for pharmaceutical protein purification, single use reagents are a preferred choice. Currently, most of recombinant protein A is produced in *E. coli* (Hammond et al., 1990, Ann. NY Acad. Sci., 613:863-867; Engel et al., 1992, Protein Expr. Purif., 3:108-113; Cai et al., 1992, Chin. J. Biotechnol., 8:93-98). Our invention allows to produce large quantities of protein A, streptococcal protein G (Bond et al., 1993, J. Immunol. Methods, 166:27-33; Du et al., 2005, Biopolymers, 79:9-17; Honda et al., 1999, Biochemistry, 38:1203-1213; their active fragments, derivatives and mimics in large quantities, said recombinant proteins being already coupled to an affinity (or chromatographic) matrix, namely the plant virus derived protein matrix. It is clearly evident from Figure 6, that CP-protein A fusion is part of recombinant viral particles containing exclusively, within the detection limits of the Coomassie stained SDS-PAGE, fusion protein (Fig. 6B) and no detectable wild type CP as building block of the viral particle.

The surface of viral particles displaying protein A serves as a high-affinity ligand suitable for purification of immunoglobulins. The relatively high molecular weight of virus particles allows their used as an affinity matrix and to develop simple procedures for purifying immunoglobulins that may be bound to the viral particles (or other protein of interest).

In addition, viral particles can be further polymerized by cross-linking yielding even higher molecular weight structure that is also suited for serving as an affinity matrix in purification procedures (Smith, Petrenko & Matthew, 1998, J. Immunol. Methods, 215:151-161). Another method of cross-linking viral particles can be by forming disulfide bridges between modified (cystein-added) coat proteins of different viral particles (Wang et al. 2002, Chem. Biol., 9: 813-819). Said approach also allows to inactivate viral particles, preventing viral vectors from replication. Additionally, different cross-linking agents can be used for inactivating viral particles, such as but not limited to formaldehyde (Barteling & Cassim, 2004, Dev. Biol., 119:449-455), ethyleneimine, N-acetylethyleneimine (Burrage et al., 2000, Vaccine, 18:2454-2461), UV irradiation (Freitas et al., 2003, J Virol Methods., 108:205-11) and other approaches.

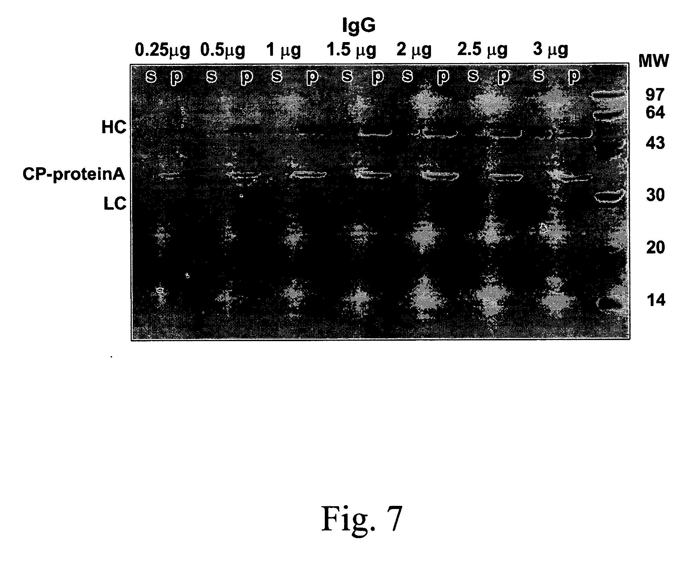

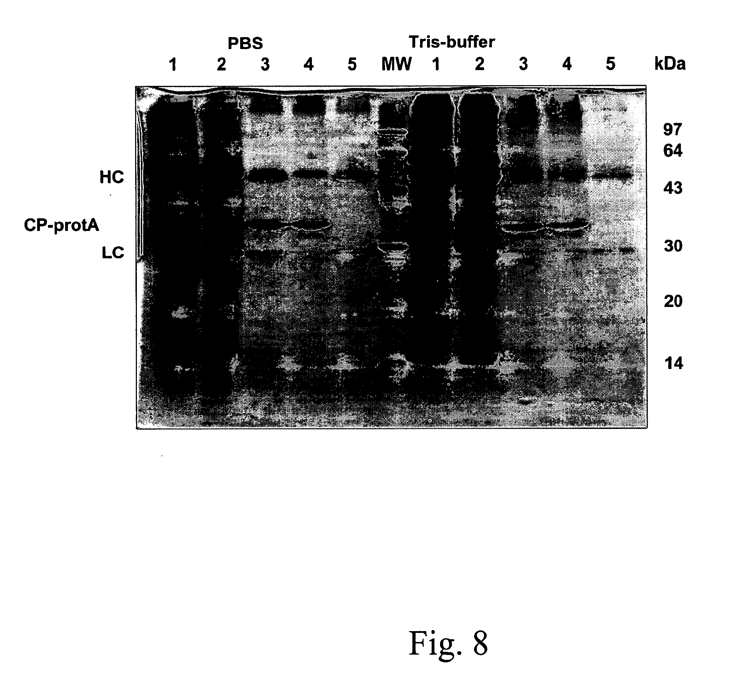

Viruses, whether naturally occurring wild-type or mutant viruses or genetically engineered viral vectors are self-replicating and as such, are very inexpensive. Plant viral particles are also much larger than the great majority of proteins or small molecules for which purification procedures are required. The great difference in molecular weight or in physico-chemical properties can be effectively exploited to separate a protein or non-proteinaceous compound of interest from a mixture such as a tissue homogenate by binding the protein of interest to a virus particle and then separating the resultant complex from the rest of the mixture. The association between the viral particle and the molecule of interest can later be dissolved in a number of ways known to those skilled in the art. In one embodiment of our invention, we demonstrate isolation of IgG from plant extract using recombinant viral particles displaying IgG-binding domains on its surface. Viral particles displaying IgG binding domains of protein A were produced and isolated as described in Example 2. After evaluation of their binding capacity (Example 3, Figure 7), said particles were used for the purification of monoclonal antibodies (IgG class) produced in *Nicotiana benthamiana* plants agroinfiltrated with non-competing viral vectors (Example 4, Figure 8). It is evident from Figure 8, that a one-step purification using said particles produces an IgG sample with ca. 95% purity. The IgG purification protocol using said particles displaying protein A as fusion with viral coat protein is summarized in Table 1.

This invention also allows generating and utilizing recombinant viral particles having on their surface more than one (two or more) recombinant proteins, thus creating complex structures on the surface of plant viral particles. This can be achieved by using recently developed plant virus based expression system permitting to express more than one CP-recombinant protein of interest fusion with high yield (EP Application No. 05 001 819.1). For example, two recombinant fusion proteins in roughly equimolar amounts can be expressed and assembled in viral particles using the invention. Alternatively, if different fusion proteins are required in a molar ratio other than equimolar, one of said fusion proteins could be expressed from a standard (e.g. driven by 35S promoter) expression cassette either transiently, or from a vector stably incorporated into plant chromosomal DNA, while the other fusion protein could be expressed from a viral vector. In yet another approach, viral particles can be reconstructed in vitro by mixing different viral particles in required proportions, deconstructing them by changing pH and/or ionic strength of the solution and then reassembling them de novo, thus producing different type of viral particles with mixture of different recombinant proteins on their surface. A schematic presentation of a plant viral particle displaying more than one recombinant protein is shown at the left, bottom, of Figure 1. Said viral particle, in addition to CP-protein A fusion, may also display a fluorescent marker (e.g. GFP or dsRed) helping to separate said viral particles or an affinity matrix thereof during a purification procedure. Additionally, a protease inhibitor protects the protein of interest to be isolated (e.g. IgG) from proteolytic degradation.

Preferably, the viral particles of the invention are produced (expressed) in plants, as plants are practically free of human and animal pathogens, thus reducing the danger of infection by using viral particles isolated from viral or bacterial source. The cost of producing viral particles in plants, plant tissue or plant cells will be significantly lower compared to viral particles produced by an animal or bacterial source. In principle, however, the method may be practiced using a wide variety of host expression systems including plants (including cell and tissue cultures thereof), animals including non-human animal organisms, and animal and human cell cultures, fungi, bacteria and yeast.

The present method of purifying proteins can be practiced in many different ways depending on several factors such as the nature of the protein of interest to be purified relative to the host and the manner in which the protein is produced in the host and the nature of the affinity between the virus and the protein. In embodiments where the protein of interest or small molecular compound to be purified is produced endogenously (naturally) by a host, the host may be cultured and lysed. The lysate or a refined solution thereof containing the protein of interest is contacted with the affinity matrix comprising the viral particles of the invention. Purification of proteins that are not produced endogenously by a host typically requires a genetic manipulation in order to supply the host with the machinery i.e., at least one transgene that encodes the protein of interest to be purified. In these embodiments, the transgene(s) may be introduced into a host as part of the viral expression/replication vector, or via a separate transformation event. The affinity of the recombinant protein displayed on the surface of the affinity matrix for the protein of interest produced by the host may be direct or indirect in the sense that the transgene may encode the protein in the form of a fusion with a binding peptide that is recognized and bound by the affinity protein on the viral particles.

In one embodiment, an exogenous (e.g., heterologous) protein of interest is expressed in a plant host (e.g., plant cells, tissue, homogenate or whole plant). This embodiment entails providing said viral particles displaying a recombinant protein as an affinity protein, wherein said recombinant protein has an affinity to the protein of interest to be purified.

Alternatively, said viral particle displays a recombinant protein that has an affinity to a small molecular compound to be purified. Therapeutic agents and herbicides are examples of such small molecular compounds. In general, any non-peptidic organic molecule produced by a host such as a plant, animal, bacterial or yeast cell, and that is recognizable (e.g. has a binding affinity for) a recombinant protein displayed on the surface of plant viral particles may be isolated or detected in accordance with the present invention. The conditions employed for dissociating the plant viral particle from the protein (or small molecule) depends on the specific type of interactions and can be created by varying physico-chemical parameters e.g., pH; temperature; ions, chelating agents concentration, etc. Selecting appropriate conditions will be within the level of skill in the art of protein purification. Ultrafiltration is one such way of separating protein from the affinity matrix of said viral particles.

This invention is suitable for the purification of transgenic and endogenous proteins of interest alike as well as non-proteinaceous molecules occurring naturally or as a consequence of transgene expression in wide variety of hosts including but not limited to members of the plant, animal and bacterial kingdoms. Examples of such proteins can be, but not limited to pharmaceutically and industrially important proteins, e.g. immune response proteins, enzymes including DNA modifying enzymes, starch-, cell wall modifying enzymes, proteases, lipases etc.

In the case of proteins or small molecular compounds that are exogenous to the host, transgenes encoding the protein of interest (by itself or in the form of a fusion with a peptide that binds the recognition peptide on the virus) or the expression of which result in the production of the small molecule, are introduced into a non-human host in accordance with standard techniques. In general, these techniques include stable or transient transformation or by way of viral delivery (e.g., infection of the cell by the viral expression vector). Methods of creating transgenic organisms with stably integrated foreign genes are well described in the literature. For example, DNA can be transformed into plant cells via Agrobacterium-mediated delivery. See, U.S. Pat. Nos.: 5,591,616; 4,940,838; and 5,464,763. Other methods include particle or microprojectile bombardment (U.S. Pat. No. 5,100,792; European Patent (EP) 444,882 B1; EP 434,616 B1), microinjection (WO 09209696; WO 09400583 A1; EP 175,966 B1) and electroporation (EP 564,595 B1; EP 290,395 B1; WO 08706614 A1). Procedures of transgene delivery into animal, bacterial and yeast cells are well established. A popular method of transgene delivery into animal cells is retrovirus-mediated (Robbins & Givizzani, 1998; Reynolds et al., 1999). Other methods with synthetic (non-viral) carriers are also suitable (for review see: Bown et al., 2001). Transformation methods for yeast and bacterial cells are well described in many manuals e.g., Yeast Protocol Handbook (2000) and Sambrook et al., (1989).

The present invention is well amenable to industrial application and scaling-up because it can accommodate techniques such as tissue homogenization, centrifugation and ultrafiltration. It can be applied to production of proteins and small molecules in any prokaryotic or eukaryotic system. Thus, the invention represents a universal, inexpensive and scale-up method of purification of any protein of interest from any kind of prokaryotic or eukaryotic system.

The viral particles of the invention can be of interest for applications in many different areas - not only in biotechnology, but also in nanotechnology and molecular electronic applications. Plant viruses are very convenient for such purposes, as they are easy to produce and isolate and provide for high yield (up to 10 g of viral particles per kilogram of fresh tobacco leaves). Also, the viral particles (virions) can be purified industrially using simple 'low-tech' protocols (Creager et al, 1999, Plant Cell, 11:301-308)

### EXAMPLES

The following examples illustrate the invention further.

### EXAMPLE 1

### Construction of TVCV- and PVX-based provectors for expression of CP-fusion proteins

The vectors used in the following examples are generally described in two recent publications (Marillonnet et al., 2004, Proc Natl Acad Sci U S A, 101:6852-6857; Marillonnet et al., 2005, Nat Biotechnol., 23:718-723). The cDNA for Potato Virus X (PVX) was generated from PVX isolate PV-0014 received from DSMZ collection (http://www.dsmz.de) by RT-PCR and used for creating PVX provectors. The descriptions of PVX-based expression system are provided in numerous publications (Chapman, Kavanagh & Baulcombe, 1992, Plant J., 2:549-557; Baulcombe, Chapman & Santa Cruz, 1995, Plant J., 7:1045-1053; Angell & Baulcombe, 1997, EMBO J., 16:3675-3684).

### a) The 5'-provectors of tobamovirus TVCV (Turnip Vein Clearing Virus)

The 3'-part of the TVCV Coat protein was amplified by PCR using primers *cptv1* and cpfus4 or *cpfus5* thus introducing a (GGGGS)₃-linker or a (EAAAK)₃-linker to the C-terminus of CP. The PCR products were cut with Ncol and Bsal and ligated into 5'-provector pICH20697 (Fig. 2A) containing the wild type CP without linker peptide resulting in constructs pICH20701 and pICH20723 (Fig. 2B). Vector pICH20697 by its intron structure is identical to that of (pICH18722) previously described (Marillonnet et al., 2005, Nature Biotechnol., 23:718-723).

### b) The 5'-provectors of PVX (Potato Virus X).

The CP with linkers was amplified by PCR with primers *pv5cptv* and *pv5p5r2* using pICH20701 or pICH20723 as template. PCR products were cloned as Nhel-Sacl fragments into PVX 5'-provector giving constructs pICH23407 and pICH23411 (Fig.2).

### c) Cloning the genes of interest in 3'-provectors

### Protein A

Protein A from *Staphylococcus aureus* contains five immunoglobulin (IgG) binding domains (Fig. 4-A). The first two of these domains (domains E and D) along with some additional amino acids on both sides (133 aa in total, Fig.4, underlined) were synthesized by GENEART (Regensburg, Germany). The sequence was optimized for expression in *Nicotiana tabacum.* The sequence was cloned as BsaI-HindIII fragment into vector plCH10990 (Marillonnet et al., 2004, Proc Natl Acad Sci U S A, 101:6852-6857) giving construct plCH21767 (Fig. 3). From this construct, the protein A sequence was transferred as HindIII-NdeI fragment into PVX 3'-provector plCH21799 resulting in plCH23523 (Fig. 3). Construct pICH21770 (not shown) is similar to plCH21767, but contains only one IgG binding domain (domain E) of protein A.

### Streptactin

Streptactin is a mutant form of streptavidin with increased affinity towards StrepTag II (Voss S. & Skerra A. 1997. Prot Engin 10, 975-982). The 5'- and 3'-part of streptactin were amplified separately by PCR using primers *streppr1* and *streppr2* or *streppr3* and *streppr4* on genomic DNA from *Streptomyces avidinii* as template. PCR products encoding for protein fragment shown in Fig. 4-B, were cut with Bsal-Bpil (*streppr1* and *streppr2*) and Bpil-BamHI (*streppr3* and *streppr4*) and ligated into 3-provector pICH10990 (Marillonnet et al., 2004, Proc Natl Acad Sci U S A, 101:6852-6857) cut with BsaI-BamHI resulting in construct pICH21444 (Fig. 3).

A mutant form of streptactin (V55T, T76R, L109T, V125R) that is supposed to be monomeric (Wu SC & Wong SL., 2005, J Biol Chem 280:23225-31) was engineered by site directed mutagensis with oligonucleotides *streppr5 -streppr12* leading to construct pICH23478 (Fig. 3).

### Strep Tag II

This tag was introduced into 3'-provector pICH21595 cut with Xbal, Bsal by adapter ligation with oligos *streptag5* and *streptag6.* The resulting construct was named pICH23463 (Fig. 3).

### Other genes

A number of other genes (GFP, DsRed, antigens, cytokines) was cloned in a similar way into 3'-provectors ( Marillonnet et al., 2004, Proc Natl Acad Sci U S A, 101:6852-6857) yielding in the constructs plCH7410 (GFP), plCH10580 (DsRED) (Fig. 3).

### EXAMPLE 2

### Expression of CP-fusions in plants

### Agroinfiltration

All constructs were electroporated into Agrobacterium tumefaciens GV3101. Agroinfiltrations of *N. benthamiana* plants were done essentially as described in Marillonnet et al., 2004, Proc Natl Acad Sci U S A, 101:6852-6857. Three agrobacterial strains containing 5' provector encoding CP, 3' provector encoding the recombinant protein and a source of a site-specific recombinase (pICH14011, Fig. 3) for assembly of viral pro-vectors *in planta* via site-specific recombination into viral vector capable of amplification and expression of recombinant protein of interest were mixed together and used for infiltration. Small-scale infiltrations were done with a syringe; large-scale infiltrations were done using a vacuum device (Marillonnet et al., 2005, Nat Biotechnol., 23:718-723).

### Analysis of CP fusions expressed in N. benthamiana leaves

All recombinant protein fusions were extracted from infiltrated *N. benthamiana* leaves 6-11 days after infiltration and analysed by electrophoretic separation in polyacrylamide gels as previously described (Marillonnet et al., 2004, Proc Natl Acad Sci U S A, 101:6852-6857; Marillonnet et al., 2005, Nat Biotechnol., 23:718-723). The results of electrophoretic analysis of different CP-recombinant protein fusions are shown in Figure 5 (A,B). It is worth noticing that direct fusion (without linker peptide) of CP with recombinant protein of interest did not produce detectable expression.

### Extraction and purification of viral particles

Infiltrated leaves were homogenized in 0.1 M K-phosphate buffer, pH 7.0 (2-3 ml buffer/g FW) using a leaf juice press or mortar and pestle. Insoluble matter was removed by filtration through miracloth. Leaf juice was treated once with one volume of chloroform and viral particles were precipitated with polyethylenglycol (PEG-6000) using standard procedures (Turpen & Reinl, 1998, Methods in Biotechnol., 3:89-101, eds. C. Cunningham & A.J.R. Porter, Humana Press, Totowa, NJ). Particles were resuspended in K-phosphate buffer and further purified by sucrose density centrifugation. The samples of viral particles containing CP fused with protein A fragment were analyzed by SDS-electrophoresis and by electronic microscopy. The samples for electron microscopy were prepared as described by Negrouk and colleagues (2004, Analytical Biochem., 333: 230-235). The results of analysis are shown in Figure 6.

### EXAMPLE 3

### Antibody binding capacity of protein A bound to the surface of plant viral particles

Aliquots (0.5 mg) of purified viral particles having bound recombinant protein A were mixed with different amounts of human IgG (Sigma 14506), incubated on ice for 1 hour and precipitated by centrifugation (10 min, 12.000 g). Pellets and supernatants were analysed by SDS-PAGE (Fig. 7). It was demonstrated that 1 mg of viral particles can bind up to 2 mg of IgG. The binding capacity is approx. 2 mg IgG/ mg of viral particles (i.e. 1 molecule of IgG (150 kDa) is bound to every 3rd - 5th molecule of CP-protein A fusion (34 kDa) on the surface of recombinant viral particle.

### EXAMPLE 4

### Purification of antibodies expressed in plants using recombinant viral particles displaying protein A on their surface

Antibodies were expressed *in planta* using ICONs viral expression system for production of hetero-oligomeric proteins in plants (EP Application No. 05 001 819.1). Leaf material containing monoclonal antibodies of the IgG class was ground in liquid nitrogen and extracted with 3 ml of PBS (Sambrook, Fritsch & Maniatis, 1989, Molecular Cloning: a Laboratory Manual, CSH, NY) per gram of fresh leaf weight (FW). Insoluble material was removed by two rounds of centrifugation (10 min, 16.000 g). One hundred milligram of viral particles displaying protein A was added per one ml of plant extract and samples were incubated on ice for at least 1 hour. Antibodies bound to viral particles were precipitated by centrifugation (15 min, 12.000 g) and resuspended in 0.25 volumes 0.1 M glycine pH 2.5. In order to remove viral particles, samples were adjusted to 1 % NaCl, 4 % PEG, incubated 30 min on ice and centrifuged 15 min at 10.000 g. Antibody-containing supernatants were transferred to fresh tubes, neutralized with 1/10 volume 1 M Tris/HCl pH 9.0 and adjusted to 14 % PEG by adding an appropriate volume of 25 % PEG-solution in PBS buffer. Samples were kept on ice for at least 1 hour and antibodies were precipitated by centrifugation (15 min, 16.000 g). Summary of purification protocol is shown in Table 1. Anbtibodies were dissolved in a convenient volume of PBS and analyzed by gel-electrophoresis. An electrophoretic analysis of proteins from purification procedure is shown in Figure 8.

**Table 1: Summary of IgG purification protocol using recombinant viral particles displaying protein A on its surface**

| | |
|---|---|
| 1. | Extract infiltrated leaves with 3 vol of PBS. Clear extract by 2x centrifugation. |
| 2. | Add viral particles (ca. 100 mg/ml) and incubate for 15-30 min on ice. |
| 3. | Centrifuge for 10 min, 12 000 g (11300 rpm in benchtop centrifuge), 4°C. |
| 4. | Discard supernatant, resuspend pellet in one volume of 0.1 M glycine, pH 2.5. |
| 5. | Add 1/10 volume of 12 % NaCl and 1/5 volume of 25 % PEG-6000 (in 0.1 M glycine, pH 2.5). Incubate on ice for 30 min. |
| 6. | Centrifuge for 15 min, 10 000 g (10300 rpm in benchtop centrifuge), 4°C. |
| 7. | Transfer supernatant to a fresh tube; neutralize with 1/10 volume of 1 M Tris/HCl pH 9.0; add 1 volume of 25 % PEG-6000 (in PBS) and incubate for 30 min on ice. |
| 8. | Centrifuge 15 min, 16 000 g (13000 rpm in benchtop centrifuge), 4°C. |
| 9. | Discard supernatant and centrifuge for additional 5 min. |
| 10 | Carefully remove all traces of supernatant; resuspend pellet in a convenient volume of PBS. |

### ANNEX 1

Primer sequences:
- *cptv1:*: 5'-cggagctcttaatttaaaagaagaaaatgtcttacaac-3'
- *cpfus4:*: 5'-tttggtctcatacctgagccaccgcctcctgatccaccgcctccacttcctccgcctc ctgtagcaggcgcagtagtcc-3'
- *pv5cptv:*: 5'-cagctagcaacaaacaagaaatgtcttacaacattacaaacccg-3'
- *pv5p5r2:*: 5'-gagctctctcgagcatgctacgcccccaactgagag-3'
- *streppr1:*: 5'-gaagacaaaggtgctgctgaggctggaattacaggcacctgg-3'
- *streppr2:*: 5'-gaagacaatgtaacataagttcctgtaagtgcaccatcggcgcc-3'
- *streppr3:*: 5'-gaagacaatacagctagaggtaatgcagaaagacgttacgtcctg-3'
- *streppr4*:: 5'-ggatccaaggtctcaacctgctgctgatggtttcaccttggtgaag-3'
- *streppr5:*: 5'-tttggtctcagagtgtaacgtctttctgcattacc-3'
- *streppr6:*: 5'-tttggtctcaactcttaccggtcgttacgacagc-3'
- *streppr7:*: 5'-tttggtctcaccctccaaccgagggcggtgcc-3'
- *streppr8:*: 5'-tttggtctcaagggtggcctggaagaataact-3'
- *streppr9:*: 5'-tttggtctcagtgtccactgggtgttgatcctcg-3'
- *streppr10*: 5'-tttggtctcaacactgacttccggcaccaccgagg-3'
- *streppr11:*: 5'-tttggtctcatctaagtgtggacttccaggcgttggc-3'
- *streppr12:*: 5'-tttgaagactatagaggacacgacaccttcaccaag-3'
- *streptag5:*: 5'-ctagcttggagtcatccacagttcgagaaataa-3'
- *streptag6*: 5'-aagcttatttctcgaactgtggatgactccaag-3'

## Claims

1. Recombinant viral particles or recombinant virus-like particles comprising a plurality of fusion protein molecules, said fusion protein comprising the following fusion protein domains:
(i) a plant viral coat protein,
(ii) a recombinant protein, and
(iii) at least one peptide linker linking said plant viral coat protein and said recombinant protein,
wherein formation of said viral particle does not require free viral coat protein.

2. Recombinant viral particles or recombinant virus-like particles comprising fusion protein molecules, said fusion protein comprising the following fusion protein segments:
(i) a plant viral coat protein,
(ii) a recombinant protein comprising at least 50 amino acid residues and
(iii) a peptide linker linking said plant viral coat protein and said recombinant protein, said peptide linker comprising at least 10 amino acid residues,
wherein said viral particle comprises at most 20 mol-% of free viral coat protein.

3. The viral particles or virus-like particles according to claim 1 or 2, wherein said viral particles are rod-shaped.

4. The viral particles or virus-like particles according to any of claims 1 to 3, wherein said viral coat protein is the coat protein of turnip vein clearing virus or a protein having an identity of at least 40 % to the coat protein of turnip vein clearing virus.

5. The viral particles or virus-like particles according to any of claims 1 to 4, wherein said plant viral coat protein has an identity of at least 60 % to the coat protein of turnip vein clearing virus.

6. The viral particles or virus-like particles according to any one of claims 1 to 5, wherein said plant viral coat protein is tobacco mosaic virus coat protein or a protein having an identity of at least 80 % to the coat protein of tobacco mosaic virus.

7. The viral particles or virus-like particles according to any of claims 1 to 6, wherein said recombinant protein is connected via said peptide linker to the N-terminal end of said plant viral coat protein.

8. The viral particles or virus-like particles according to any of claims 1 to 6, wherein said recombinant protein is connected via said peptide linker to the C-terminal end of said plant viral coat protein.

9. The viral particles or virus-like particles according to any of claims 1 to 6, wherein said recombinant protein is an internal fusion with respect to said plant viral coat protein and is connected via two peptide linkers to said plant viral coat protein.

10. The viral particles or virus-like particles according to any of claims 1 to 9, wherein said linker peptide has no defined secondary structure or forms a helix.

11. The viral particles or virus-like particles according to any of claims 1 to 10, wherein said viral particles display two or more different recombinant proteins on their surface.

12. The viral particles or virus-like particles according to any of claims 1 to 11, wherein said recombinant protein has affinity to immunoglobulins or derivatives thereof.

13. The viral particles or virus-like particles according to claim 12, wherein said recombinant protein is staphylococcal protein A or a derivative thereof capable of binding immunoglobulins.

14. The viral particles or virus-like particles according to claim 12, wherein said recombinant protein is streptococcal protein G or a derivative thereof capable of binding immunoglobulins.

15. The viral particles or virus-like particles according to claim 12, wherein said recombinant protein is streptavidin or a derivative thereof.

16. The viral particles or virus-like particles of any of claims 1 to 15, wherein viral particle comprises at most 20 mol%, preferably at most 10 mol-%, of free viral coat protein.

17. Recombinant viral particles or recombinant virus-like particles comprising fusion protein molecules, said fusion protein comprising the following fusion protein domains:
(i) a plant viral coat protein of turnip vein clearing virus or a protein having an identity of at least 40 % to the coat protein of turnip vein clearing virus,
(ii) a recombinant protein comprising at least 50 amino acid residues and
(iii) a peptide linker linking said plant viral coat protein and said recombinant protein, said peptide linker comprising at least 10 amino acid residues.

18. A process of purifying a protein of interest, comprising the following steps:
(a) providing an affinity matrix comprising a fusion protein as defined in any of claims 1 to 17, said recombinant protein of said fusion protein having affinity to said protein of interest to be purified,
(b) contacting said affinity matrix with a liquid composition containing said protein of interest under conditions allowing binding of said protein of interest to said recombinant protein of said affinity matrix, whereby said affinity matrix is insoluble under said conditions,
(c) removing components of said liquid composition that have not bound to said recombinant protein from the mixture of step (b) under conditions preserving binding of said protein of interest to said recombinant protein of said affinity matrix, and
(d) separating said protein of interest from said affinity matrix.

19. The process according to claim 18, wherein said recombinant protein has affinity to immunoglobulins or derivatives thereof, preferably said affinity protein is staphylococcal protein A or a derivative thereof.

20. The process according to claim 18, wherein said affinity matrix comprises recombinant viral particles or recombinant plant virus-like particles, said affinity matrix containing at most 20 mol-% of free viral coat protein relative to the sum of free viral coat protein and said fusion protein present in said affinity matrix.

21. A process of producing recombinant viral particles or recombinant virus-like particles as defined in any of claims 1 to 17, comprising expressing said fusion protein in a bacterium, in a plant, in plant tissue, or in plant cells.

22. The process according to claim 21, comprising introducing a plant viral vector encoding said fusion protein into a plant cell by agrobacterium-mediated delivery, followed by isolating said viral particles from said plant cell.

23. The process according to claim 21 or 22, further comprising rendering said viral particles infection-deficient using chemical inactivation.

24. A process of producing recombinant viral particles or recombinant virus-like particles, comprising assembling recombinant viral particles in a mixture comprising fusion protein as defined in any of claims 1 to 17 and a second protein being or comprising a coat protein under conditions allowing assembly of viral particles comprising said fusion protein and said second protein.

25. A fusion protein comprising the following fusion protein segments: a plant viral coat protein, a linker peptide, and a recombinant protein capable of binding immunoglobulins, whereby said fusion protein is capable of forming recombinant viral particles or virus-like particles.

26. A polynucleotide encoding the fusion protein defined in any of claims 1 to 17 or 25.

27. A plant, plant tissue or plant cells comprising the polynucleotide of claim 26.

28. Viral material obtained or obtainable from the plant, the plant tissue or plant cells of claim 27.

29. Kit-of-parts comprising a plant, plant tissue or plant cells and a polynucleotide as defined in claim 25.

30. A affinity matrix for affinity purification of a protein of interest, said protein matrix comprising recombinant viral particles or recombinant virus-like particles comprising fusion protein molecules as defined in any of claims 1 to 17, wherein said viral particles comprise at most 20 mol-% of free viral coat protein relative to the sum of free viral coat protein and said fusion protein present in said affinity matrix.

31. The protein matrix of claim 30, said protein matrix comprising said viral particles or virus-like particles that are chemically cross-linked.
